(19) Europäisches Patentamt
European Patent Office
Office européen des brevets

(11) EP 3 701 806 A1

(12) EUROPEAN PATENT APPLICATION

(43) Date of publication:
02.09.2020 Bulletin 2020/36

(21) Application number: 19160264.8

(22) Date of filing: 01.03.2019

(51) Int Cl.:
*A23L 27/00* (2016.01) *A23L 27/60* (2016.01)
*A23L 33/105* (2016.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **DuPont Nutrition Biosciences ApS**
**1411 Copenhagen K (DK)**

(72) Inventors:
- **BARON, Christopher**
**New Century, Kansas 66031 (US)**
- **ZHONG, Ying J.**
**New Century, Kansas 66031 (US)**

(74) Representative: **DuPont EMEA**
**Langebrogade 1**
**1411 Copenhagen K (DK)**

# (54) COMPOSITION AND USE THEREOF IN AN EMULSION OR A FOODSTUFF

(57) The present disclosure relates to a composition that exhibits an antioxidant action. The present disclosure also relates to an emulsion or a foodstuff comprising the composition and to the use of the composition for preventing and/or inhibiting oxidation of an emulsion or a foodstuff. Moreover, the present disclosure relates to a process for preventing and/or inhibiting oxidation of an emulsion or a foodstuff comprising contacting a foodstuff or an emulsion with the composition. The present disclosure also relates to a composition that can impart flavour, and to the use of the composition for imparting flavour to an emulsion or a foodstuff.

EP 3 701 806 A1

## Description

### FIELD

The present disclosure relates to a composition that exhibits an antioxidant action. The present disclosure also relates to an emulsion or a foodstuff comprising the composition and to the use of the composition for preventing and/or inhibiting oxidation of an emulsion or a foodstuff. Moreover, the present disclosure relates to a process for preventing and/or inhibiting oxidation of an emulsion or a foodstuff comprising contacting a foodstuff or an emulsion with the composition. The present disclosure also relates to a composition that can impart flavour, and to the use of the composition for imparting flavour to an emulsion or a foodstuff.

### BACKGROUND

Antioxidants are widely used in food products susceptible to oxidative degeneration. An antioxidant is defined by the Food and Drug Administration (21CFR 170.3) as "a substance used to preserve food by retarding deterioration, rancidity, or discoloration due to oxidation". There is an increasing need to develop economical, natural and effective food preservative systems to meet the public demand for convenient, natural, safe, healthy, good quality food products with guaranteed shelf life. To this end spices or plant extracts can be used in food as antioxidants and to impart flavour. One advantage of such extracts is that they are perceived as natural ingredients when compared to antioxidants such as ethylenediaminetetraacetic acid (EDTA), butyl hydroxyanisol (BHA) and butylated hydroxytoluene (BHT).

There is large number of antioxidants known based on naturally occurring plant materials. It is noted that these materials have varying degrees of efficacy. Moreover, the antioxidant levels required to ensure preservation safety may prove uneconomical, or are above levels acceptable due to regulatory and legislation constraints when present in amounts sufficient to offer the required protection. In addition, the antioxidant level required can have an undesirable sensory impact.

Many known antioxidants may be readily dispersed in water based foodstuffs but are only sparingly soluble in oil based foodstuffs. This is problematic as it limits the range of foods or feeds in which the products may be used. In oil based foodstuffs, the antioxidant may be poorly dispersed resulting in sub-optimal performance. Similar disadvantages may be observed in foods and feeds containing water and oil.

The present disclosure alleviates the problems of the prior art.

### SUMMARY

This specification discloses, in part, a composition comprising:

(a) a green tea [*Camellia sinensis*] extract; and
(b) a date [*Phoenix dactylifera*] product,

wherein the date product is selected from date powder, date fermentate, date extract and mixtures thereof.

This specification also discloses, in part, an emulsion comprising a composition comprising:

(a) a green tea [*Camellia sinensis*] extract; and
(b) a date [*Phoenix dactylifera*] product,

wherein the date product is selected from date powder, date fermentate, date extract and mixtures thereof.

This specification also discloses, in part, a foodstuff comprising a composition comprising:

(a) a green tea [*Camellia sinensis*] extract; and
(b) a date [*Phoenix dactylifera*] product,

wherein the date product is selected from date powder, date fermentate, date extract and mixtures thereof.

This specification also discloses, in part, a process for preventing and/or inhibiting oxidation of a foodstuff or an emulsion, comprising contacting a foodstuff with a composition comprising:

(a) a green tea [*Camellia sinensis*] extract; and
(b) a date [*Phoenix dactylifera*] product,

wherein the date product is selected from date powder, date fermentate, date extract and mixtures thereof.

This specification also discloses, in part, use of a composition comprising:

(a) a green tea [*Camellia sinensis*] extract; and
(b) a date [*Phoenix dactylifera*] product,

wherein the date product is selected from date powder, date fermentate, date extract and mixtures thereof for preventing or inhibiting oxidation of an emulsion or a foodstuff.

Aspects of the disclosure are defined in the appended claims.

The present disclosure provides, in part, a combination of components for preventing and/or inhibiting oxidation in a material, such as a foodstuff. The present disclosure also provides a synergistic combination of components for preventing and/or inhibiting oxidation in a material, such as a foodstuff. This combination of components allows lower levels of the antioxidants to be used to provide effective action. This is particularly important in food applications where reduction of dosage is desired for commercial and regulatory reasons.

By the term 'antioxidant' it is meant a substance which reduces the amount of oxidation over a given period when compared to the oxidation that would occur in the absence of that substance or it is a meant a material which increase the time required for a given amount of oxidation to occur when compared to the oxidation that would occur in the absence of that substance.

The present disclosure also provides, in part, a combination of components for imparting flavour to a material, such as a foodstuff.

By the term "flavourant" it is meant a substance which alters or enhances the taste of a material when compared to the taste of a material in the absence of that substance.

For ease of reference, these and further disclosures are now discussed under appropriate section headings. However, the teachings under each section are not necessarily limited to each particular section.

## DETAILED DESCRIPTION

### *Green Tea Extract*

As discussed in this specification one component in the composition of the present disclosure is green tea [*Camellia sinensis*] extract. All references to green tea extract in this specification mean an extract from a plant of the species *Camellia sinensis.*

By the term "extract" or "extracts" it is meant any constituent of the plant which may be isolated from the whole plant.

In some embodiments, by the term green tea "extract" or "extracts" of it is meant a leaf of the plant or a constituent which may be isolated from the leaf of the whole plant. In other embodiments, by the term green tea "extract" or "extracts" of it is meant a bud of the plant or a constituent which may be isolated from the bud of the whole plant.

Green tea extracts are soluble in water and insoluble in oil. Numerous *in vitro* and *in vivo* studies have suggested the beneficial health properties of green tea (*Camellia sinensis*) and tea polyphenols including antioxidant and antimicrobial activity [Kubo et al]. To summarise, catechins (flavonoids) are a major component of green tea. According to Yam *et. al.* (1997) catechins and their gallates are the main chemical moieties responsible for the antimicrobial activity of green tea extracts with the substances having *epi-* configuration being seemingly more active [Kajiya et al]. In green tea extracts the antioxidant activity is primarily due to four catechins, of which the main components are Epigallocatechin gallate (EGCg) and Epicatechin gallate (ECg).

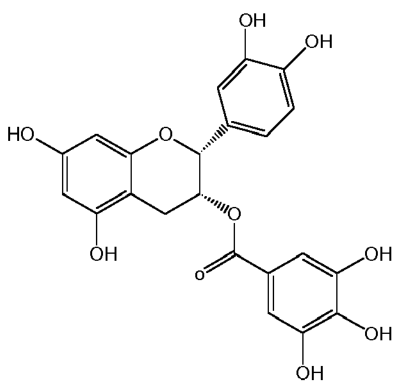

(-)-Epicatechin gallate

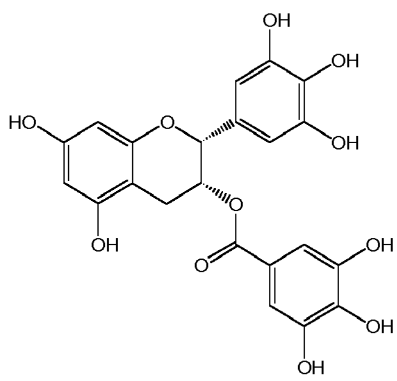

(-)-Epigallocatechin gallate

In some embodiments of the present disclosure the composition comprises at least an antioxidant selected from green tea [*Camellia sinensis*] extract.

In some embodiments of the present disclosure the green tea extract is a tea polyphenol. In some embodiments of the disclosure the green tea extract is a catechin. In some embodiments of the present disclosure the green tea extract is a compound selected from:

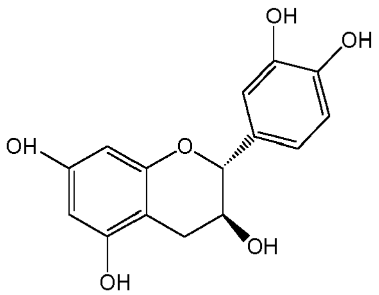

(+)-Catechin,

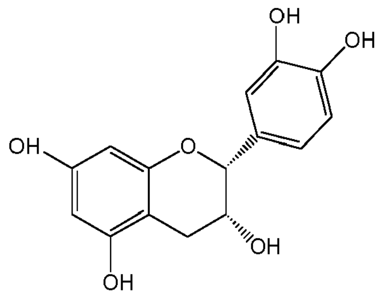

(-)-Epicatechin,

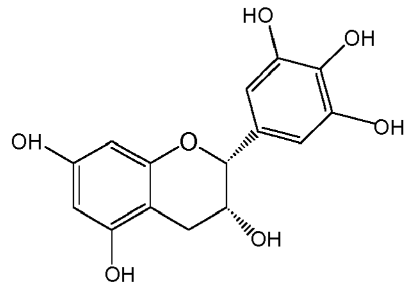

(-)-Epigallocatechin,

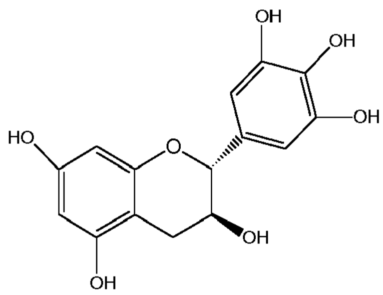

(+)-Gallocatechin,

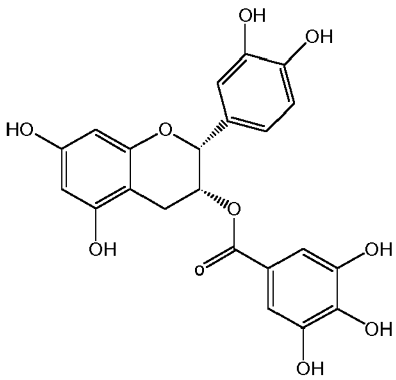

(-)-Epicatechin gallate,

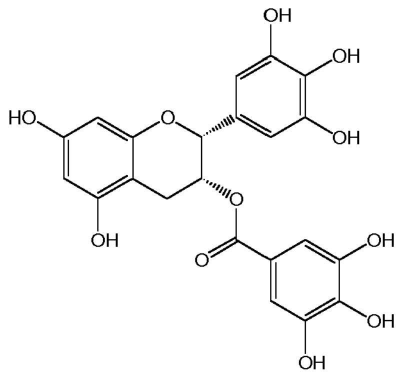

(-)-Epigallocatechin gallate,

and mixtures thereof. These six compounds are referred to in this specification as green tea catechins or green tea extract catechins. Green tea extract may contain these six catechins in a combined amount of approximately 75wt.% based on the green tea extract.

In some embodiments of the present disclosure, the green tea extract is a compound selected from:

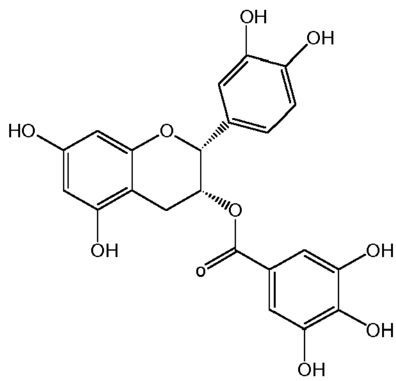

(-)-Epicatechin gallate,

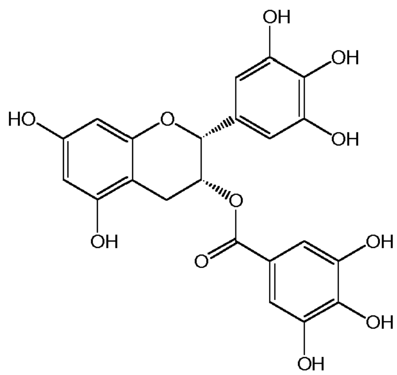

(-)-Epigallocatechin gallate,

and mixtures thereof.

The green tea extract may be present in any amount to provide the required antioxidant effect. The green tea extract may be present in any amount to impart the required flavour.

The amounts of green tea extract are typically denoted by the amount of catechins provided by the green tea

extract. Suitable amounts of green tea extract for use in the present disclosure are given below. In some embodiments the amounts of green tea extract recited in this specification are based on a green tea extract containing 75wt.% catechins. In these embodiments, one skilled in the art could modify the amounts recited in this specification should the green tea extract being used contain catechins in an amount of greater or less than 75wt.%.

In some embodiments, the composition comprises the green tea extract in an amount of at least 0.1 wt.% based on the total weight of the composition. In some embodiments, the composition comprises the green tea extract in an amount of at least 0.2 wt.% based on the total weight of the composition. In some embodiments, the composition comprises the green tea extract in an amount of at least 0.5 wt.% based on the total weight of the composition. In some embodiments, the composition comprises the green tea extract in an amount of at least 1 wt.% based on the total weight of the composition. In some embodiments, the composition comprises the green tea extract in an amount of at least 2 wt.% based on the total weight of the composition. In some embodiments, the composition comprises the green tea extract in an amount of at least 3 wt.% based on the total weight of the composition. In some embodiments, the composition comprises the green tea extract in an amount of at least 4 wt.% based on the total weight of the composition. In some embodiments, the composition comprises the green tea extract in an amount of at least 5 wt.% based on the total weight of the composition.

In some embodiments, the composition comprises the green tea extract in an amount of no greater than 40 wt.% based on the total weight of the composition. In some embodiments, the composition comprises the green tea extract in an amount of no greater than 35 wt.% based on the total weight of the composition. In some embodiments, the composition comprises the green tea extract in an amount of no greater than 30 wt.% based on the total weight of the composition. In some embodiments, the composition comprises the green tea extract in an amount of no greater than 25 wt.% based on the total weight of the composition. In some embodiments, the composition comprises the green tea extract in an amount of no greater than 20 wt.% based on the total weight of the composition. In some embodiments, the composition comprises the green tea extract in an amount of no greater than 15 wt.% based on the total weight of the composition. In some embodiments, the composition comprises the green tea extract in an amount of no greater than 12 wt.% based on the total weight of the composition. In some embodiments, the composition comprises the green tea extract in an amount of no greater than 11 wt.% based on the total weight of the composition. In some embodiments, the composition comprises the green tea extract in an amount of no greater than 10 wt.% based on the total weight of the composition. In some embodiments, the composition comprises the green tea extract in an amount of no greater than 8 wt.% based on the total weight of the composition. In some embodiments, the composition comprises the green tea extract in an amount of no greater than 6 wt.% based on the total weight of the composition. In some embodiments, the composition comprises the green tea extract in an amount of no greater than 5 wt.% based on the total weight of the composition.

In some embodiments, the composition comprises the green tea extract in an amount of from 0.1 to 20 wt.% based on the total weight of the composition. In some embodiments, the composition comprises the green tea extract in an amount of from 0.2 to 20 wt.% based on the total weight of the composition. In some embodiments, the composition comprises the green tea extract in an amount of from 0.5 to 20 wt.% based on the total weight of the composition. In some embodiments, the composition comprises the green tea extract in an amount of from 1 to 20 wt.% based on the total weight of the composition. In some embodiments, the composition comprises the green tea extract in an amount of from 2 to 20 wt.% based on the total weight of the composition. In some embodiments, the composition comprises the green tea extract in an amount of from 3 to 20 wt.% based on the total weight of the composition. In some embodiments, the composition comprises the green tea extract in an amount of from 4 to 20 wt.% based on the total weight of the composition, to 20 wt.%

In some embodiments, the composition comprises the green tea extract in an amount of from 0.1 to 18 wt.% based on the total weight of the composition. In some embodiments, the composition comprises the green tea extract in an amount of from 0.1 to 16 wt.% based on the total weight of the composition. In some embodiments, the composition comprises the green tea extract in an amount of from 0.1 to 14 wt.% based on the total weight of the composition. In some embodiments, the composition comprises the green tea extract in an amount of from 0.1 to 12 wt.% based on the total weight of the composition. In some embodiments, the composition comprises the green tea extract in an amount of from 0.1 to 10 wt.% based on the total weight of the composition. In some embodiments, the composition comprises the green tea extract in an amount of from 0.1 to 8 wt.% based on the total weight of the composition. In some embodiments, the composition comprises the green tea extract in an amount of from 0.1 to 6 wt.% based on the total weight of the composition. In some embodiments, the composition comprises the green tea extract in an amount of from 0.1 to 5 wt.% based on the total weight of the composition.

In some embodiments, the composition comprises the green tea extract in an amount of from 0.1 to 15 wt.% based on the total weight of the composition. In some embodiments, the composition comprises the green tea extract in an amount of from 0.2 to 15 wt.% based on the total weight of the composition. In some embodiments, the composition comprises the green tea extract in an amount of from 0.5 to 15 wt.% based on the total weight of the composition. In some embodiments, the composition comprises the green tea extract in an amount of from 1 to 15 wt.% based on the

total weight of the composition. In some embodiments, the composition comprises the green tea extract in an amount of from 2 to 15 wt.% based on the total weight of the composition. In some embodiments, the composition comprises the green tea extract in an amount of from 3 to 15 wt.% based on the total weight of the composition. In some embodiments, the composition comprises the green tea extract in an amount of from 4 to 15 wt.% based on the total weight of the composition. to 15 wt.% to 15 wt.% to 15 wt.%

In some embodiments, the composition comprises the green tea extract in an amount of from 1 to 18 wt.% based on the total weight of the composition. In some embodiments, the composition comprises the green tea extract in an amount of from 1 to 16 wt.% based on the total weight of the composition. In some embodiments, the composition comprises the green tea extract in an amount of from 1 to 14 wt.% based on the total weight of the composition. In some embodiments, the composition comprises the green tea extract in an amount of from 1 to 12 wt.% based on the total weight of the composition. In some embodiments, the composition comprises the green tea extract in an amount of from 1 to 10 wt.% based on the total weight of the composition. In some embodiments, the composition comprises the green tea extract in an amount of from 1 to 8 wt.% based on the total weight of the composition. In some embodiments, the composition comprises the green tea extract in an amount of from 1 to 6 wt.% based on the total weight of the composition. In some embodiments, the composition comprises the green tea extract in an amount of from 1 to 5 wt.% based on the total weight of the composition.

In some embodiments, the composition comprises the green tea extract in an amount of from 0.1 to 10 wt.% based on the total weight of the composition. In some embodiments, the composition comprises the green tea extract in an amount of from 0.2 to 10 wt.% based on the total weight of the composition. In some embodiments, the composition comprises the green tea extract in an amount of from 0.5 to 10 wt.% based on the total weight of the composition. In some embodiments, the composition comprises the green tea extract in an amount of from 1 to 10 wt.% based on the total weight of the composition. In some embodiments, the composition comprises the green tea extract in an amount of from 2 to 10 wt.% based on the total weight of the composition. In some embodiments, the composition comprises the green tea extract in an amount of from 3 to 10 wt.% based on the total weight of the composition. In some embodiments, the composition comprises the green tea extract in an amount of from 4 to 10 wt.% based on the total weight of the composition.

In some embodiments, the composition comprises the green tea extract in an amount of from 4 to 18 wt.% based on the total weight of the composition. In some embodiments, the composition comprises the green tea extract in an amount of from 4 to 16 wt.% based on the total weight of the composition. In some embodiments, the composition comprises the green tea extract in an amount of from 4 to 14 wt.% based on the total weight of the composition. In some embodiments, the composition comprises the green tea extract in an amount of from 4 to 12 wt.% based on the total weight of the composition. In some embodiments, the composition comprises the green tea extract in an amount of from 4 to 10 wt.% based on the total weight of the composition. In some embodiments, the composition comprises the green tea extract in an amount of from 4 to 8 wt.% based on the total weight of the composition. In some embodiments, the composition comprises the green tea extract in an amount of from 4 to 6 wt.% based on the total weight of the composition. In some embodiments, the composition comprises the green tea extract in an amount of from 4 to 5 wt.% based on the total weight of the composition.

In some embodiments, the composition comprises the green tea extract in an amount of from 1.5 to 7 wt.% based on the total weight of the composition. In some embodiments, the composition comprises the green tea extract in an amount of from 2 to 7 wt.% based on the total weight of the composition. In some embodiments, the composition comprises the green tea extract in an amount of from 4 to 7 wt.% based on the total weight of the composition.

In some embodiments, the composition comprises the green tea extract in an amount of from 0.1 to 5 wt.% based on the total weight of the composition. In some embodiments, the composition comprises the green tea extract in an amount of from 0.2 to 5 wt.% based on the total weight of the composition. In some embodiments, the composition comprises the green tea extract in an amount of from 0.5 to 5 wt.% based on the total weight of the composition. In some embodiments, the composition comprises the green tea extract in an amount of from 1 to 5 wt.% based on the total weight of the composition. In some embodiments, the composition comprises the green tea extract in an amount of from 2 to 5 wt.% based on the total weight of the composition. In some embodiments, the composition comprises the green tea extract in an amount of from 3 to 5 wt.% based on the total weight of the composition. In some embodiments, the composition comprises the green tea extract in an amount of from 4 to 5 wt.% based on the total weight of the composition.

In some embodiments, the composition comprises green tea extract catechins, namely

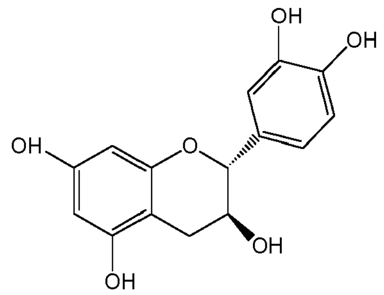
(+)-Catechin,

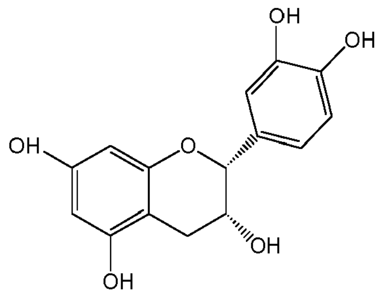
(-)-Epicatechin,

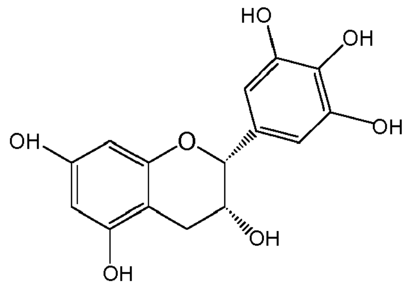
(-)-Epigallocatechin,

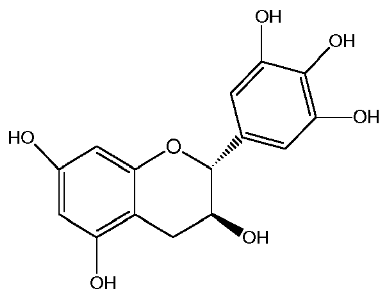
(+)-Gallocatechin,

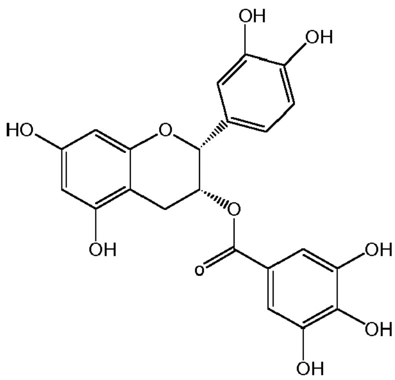
(-)-Epicatechin gallate, and

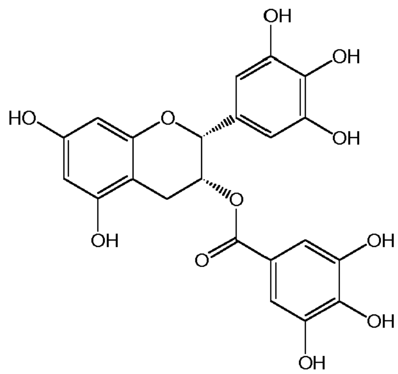
(-)-Epigallocatechin gallate

in a combined amount of at least 0.1 wt.% based on the composition. The green tea extract catechins may be present in a combined amount of at least 0.2 wt.% based on the composition. The green tea extract catechins may be present in a combined amount of at least 0.3 wt.% based on the composition. The green tea extract catechins may be present in a combined amount of at least 0.4 wt.% based on the composition. The green tea extract catechins may be present in a combined amount of at least 0.5 wt.% based on the composition. The green tea extract catechins may be present in a combined amount of at least 1.0 wt.% based on the composition. The green tea extract catechins may be present in a combined amount of at least 1.5 wt.% based on the composition. Yet further the green tea extract catechins may be present in a combined amount of at least 2.0 wt.% based on the composition.

The composition may comprise the green tea extract catechins in a combined amount of 0.1 to 10 wt.% based on the composition. The green tea extract catechins may be present in a combined amount of 0.2 to 10 wt.% based on the composition. The green tea extract catechins may be present in a combined amount of 0.5 to 10 wt.% based on the composition. The green tea extract catechins may be present in a combined amount of 1 to 10 wt.% based on the composition. The green tea extract catechins may be present in a combined amount of 1.5 to 7 wt.% based on the composition. The green tea extract catechins may be present in a combined amount of 2 to 7 wt.% based on the composition. The green tea extract catechins may be present in a combined amount of 4 to 7 wt.% based on the composition.

***Date Product***

As discussed in this specification one component in the composition of the present disclosure is a date [*Phoenix dactylifera*] product, which is selected from date powder, date fermentate, date extract and mixtures thereof. All references in this specification to date product mean a product from the *Phoenix dactylifera,* commonly known as the date palm.

By the term "powder" it is meant the whole fruit or any part thereof that has been dried and ground.

By the term "extract" or "extracts" it is meant any constituent of the fruit which may be isolated from the whole fruit.

By the term "fermentate" it is meant the partial or complete product of date fermentation.

Dates are single pitted fruits with three main parts: date flesh, date pit, and date skin. The predominant components in dates are carbohydrates, which includes soluble sugars and dietary fibre. Dates also include a variety of phenolic compounds, which reportedly exhibit antioxidant properties [Ghnimi et al.]. The following phenolic compounds have been identified in dates: gallic acid, protocatechuic acid, *p*-hydroxybenzoic acid, vanillic acid, sinapic acid, syringic acid, caffeic acid, hydrocaffeic acid, ferulic acid, *p*-coumaric acid, syringic acid, dactyliferic acid, 2 caffeoylshikimic acid

hexosides, 3-caffeoylshikimic acid, 4-caffeoylshikimic acid, 5-caffeoylshikimic acid, caffeoylsinapoyl hexoside, dicaffeoylsinapoyl hexoside, luteolin, quercetin and apigenin, quercetin rhamnosyl-hexoside sulfate, quercetin 3-O-rutinoside (rutin), quercetin hexoside sulfate, quercetin acetyl-hexoside, isorhamnetin-3-O-rutinoside, isorhamnetin hexoside, chrysoeriol rhamnosyl-hexoside, isorhamnetin acetyl-hexoside, quercetin 3-O-glucoside (isoquercitrin), chrysoeriol hexoside sulfate, chrysoeriol hexoside, (+)-catechin, and (-)-epicatechin, procyanidin oligomers based on (-)-epicatechin including procyanidin B1, procyanidin B2, procyanidin trimer, procyanidin tetramer, procyanidin pentamer, procyanidin polymers based on (-)-epicatechin (decemers to heptadecemers) and cyaniding [Ghnimi et al.]. It is noted that the proanthocyanidins (also known as date tannins) are the major phenols in the edible parts of ripe dates amounting to around 1.5% and representing about 95% of total polyphenols.

There are many varieties of date including, but not limited to: *Aabel, Ajwah, Al-Khunaizi, Amir Hajj (or Amer Hajj), Abid Rahim, Barakawi, Barhee (or Barhi), Bireir, Dabbas, Datça, Deglet Noor, Derrie (or Dayri), Empress, Fardh (or Fard), Ftimi (or Alligue), Holwah (or Halawi), Haleema, Hayany (or Hayani), Iteema, Kenta, Khadrawi (or Khadrawy), Khalasah, Khastawi (or Khusatawi or Kustawy), Khenaizi, Lulu, Maktoom, Manakbir, Medjool (or Majdool), Migraf (or Mejraf), Mgmaget Ayuob, Mishriq, Mazafati (or Mozafati), Nabtat-seyf, Piarom, Rotab, Sag'ai, Saidy (or Saidi), Sayer (or Sayir), Sukkary, Sellaj, Indi, Tagyat, Tamej, Thoory (or Thuri), Umeljwary, Umelkhashab, Zahidi, Zaghloul.* Dates develop through five different stages of ripening: Hanabauk (1-5 weeks from pollination), Kimri (the following 9-14 weeks), Khalal (or Bisr) (the following 6 weeks), Rutab (the following 4 weeks), and Tamr (the last two weeks). The fruits become edible in the final three stages as a result of decreased bitterness, increased sweetness, and improved tenderness, and succulence.

Any suitable variety of date may be used in the present disclosure. It is possible to use dates at all stages of ripening in the present disclosure.

In some embodiments, the date [*Phoenix dactylifera*] product is date powder. In some embodiments, the date powder is prepared from the whole fruit. In some embodiments, the date powder is prepared from the date skin and date flesh. In some embodiments, the date powder is prepared from the whole fruit minus the pit. In some embodiments, the date powder is prepared from the date pit. In some embodiments, the date powder is prepared from the whole date minus the skin. In some embodiments, the date powder is prepared from the date skin. In some embodiments, the date powder is prepared from the date flesh.

In some embodiments, the date [*Phoenix dactylifera*] product is date fermentate.

In some embodiments, the date [*Phoenix dactylifera*] product is date extract. In some embodiments, by the term date "extract" or "extracts" of it is meant the skin, the seed or the flesh of the date or any constituent which may be isolated from the skin, the seed or the flesh of a date. In some embodiments, the date extract is date skin. In some embodiments, the date extract is date flesh. In some embodiments, the date extract is the date seed.

In some embodiments, the date extract is date juice. The date juice may be from any part of the date. In some embodiments, the date juice is from the date flesh and date skin. In some embodiments, the date juice is from the date flesh.

In some embodiments, the date extract is date juice concentrate. It will be appreciated by one skilled in the art that by the term "date fruit concentrate" it is meant date juice that has been dried.

In some embodiments, the date [*Phoenix dactylifera*] product is a mixture of date powder, date extract and date fermentate. In some embodiments, the date [*Phoenix dactylifera*] product is a mixture of date powder and date fermentate. In some embodiments, the date [*Phoenix dactylifera*] product is a mixture of date powder and date extract. In some embodiments, the date [*Phoenix dactylifera*] product is a mixture of date extract and date fermentate.

In some embodiments, the composition comprises the date product in an amount of at least 10 wt.% based on the composition. In some embodiments, the composition comprises the date product in an amount of at least 20 wt.% based on the composition. In some embodiments, the composition comprises the date product in an amount of at least 30 wt.% based on the composition. In some embodiments, the composition comprises the date product in an amount of at least 40 wt.% based on the composition. In some embodiments, the composition comprises the date product in an amount of at least 50 wt.% based on the composition. In some embodiments, the composition comprises the date product in an amount of at least 60 wt.% based on the composition. In some embodiments, the composition comprises the date product in an amount of at least 70 wt.% based on the composition. In some embodiments, the composition comprises the date product in an amount of at least 75 wt.% based on the composition.

In some embodiments, the composition comprises the date product in an amount of no greater than 99 wt.% based on the composition. In some embodiments, the composition comprises the date product in an amount of no greater than 95 wt.% based on the composition. In some embodiments, the composition comprises the date product in an amount of no greater than 90 wt.% based on the composition. In some embodiments, the composition comprises the date product in an amount of no greater than 85 wt.% based on the composition. In some embodiments, the composition comprises the date product in an amount of no greater than 80 wt.% based on the composition. In some embodiments, the composition comprises the date product in an amount of no greater than 75 wt.% based on the composition.

In some embodiments, the composition comprises the date product in an amount of from 40 to 90 wt.% based on the composition. In some embodiments, the composition comprises the date product in an amount of from 40 to 85

wt.% based on the composition. In some embodiments, the composition comprises the date product in an amount of from 40 to 80 wt.% based on the composition. In some embodiments, the composition comprises the date product in an amount of from 40 to 75 wt.% based on the composition.

In some embodiments, the composition comprises the date product in an amount of from 50 to 90 wt.% based on the composition. In some embodiments, the composition comprises the date product in an amount of from 50 to 85 wt.% based on the composition. In some embodiments, the composition comprises the date product in an amount of from 50 to 80 wt.% based on the composition. In some embodiments, the composition comprises the date product in an amount of from 50 to 75 wt.% based on the composition.

### *Composition*

The composition described in this specification can be an antioxidant composition. The composition described in this specification can be a flavourant composition.

The components of the composition utilised in the present disclosure may be present in any amount to provide an antioxidant effect and in particular (a) a green tea [*Camellia sinensis*] extract; and (b) a date [*Phoenix dactylifera*] product selected from date powder, date fermentate, date extract and mixtures thereof, are present in amounts to provide an antioxidant effect, such as a synergistic antioxidant effect.

The components of the composition utilised in the present disclosure may be present in any amount to provide a desired flavour.

In some embodiments the weight ratio of (a) green tea [*Camellia sinensis*] extract, to (b) date [*Phoenix dactylifera*] product, is from 30:1 to 1 :30, such as 30:1 to 1:1, such as 1:1 to 1:30, such as 25:1 to 1:25. such as 25:1 to 1:1, such as 1:1 to 1:25, such as 20:1 to 1:20, such as 20:1 to 1:1, such as 1:1 to 1:20, such as 15:1 to 1:15, such as 15:1 to 1:1, such as 1:1 to 1:15, such as 10:1 to 1:10, such as 10:1 to 1:1, such as 1:1 to 1:10, such as 5:1 to 1:5, such as 5:1 to 1:1, such as 1:1 to 1:5, such as 4:1 to 1:4, such as 4:1 to 1:1, such as 1:1 to 1:4.

In some embodiments the weight ratio of (a) green tea [*Camellia sinensis*] extract, to (b) date [*Phoenix dactylifera*] product, is from 4:1 to 1:10, such as 4:1 to 1:8, such as 4:1 to 1:5 such as 4: 1 to 1:4. In some embodiments the weight ratio of (a) green tea [*Camellia sinensis*] extract, to (b) date [*Phoenix dactylifera*] product, is from 2: 1 to 1:10, such as 2:1 to 1:8, such as 2:1 to 1:5 such as 2:1 to 1:4. In some embodiments the weight ratio of (a) green tea [*Camellia sinensis*] extract, to (b) date [*Phoenix dactylifera*] product, is from 1:1 to 1:10, such as 1:1 to 1:8, such as 1:1 to 1:5 such as 1:1 to 1:4. In other embodiments the weight ratio of (a) green tea [*Camellia sinensis*] extract, to (b) date [*Phoenix dactylifera*] product, is from 4:1 to 1:5, such as 2:1 to 1:5, such as 1:1 to 1:5. In other embodiments the weight ratio of (a) green tea [*Camellia sinensis*] extract, to (b) date [*Phoenix dactylifera*] product, is from 4:1 to 1:4, such as 2:1 to 1:4, such as 1:1 to 1:4.

In some embodiments the weight ratio of (a) green tea [*Camellia sinensis*] extract, to (b) date [*Phoenix dactylifera*] product, is from 1:1 to 1:10, such as 1:1 to 1:8, such as 1:1 to 1:5 such as 1:2 to 1:5. In some embodiments the weight ratio of (a) green tea [*Camellia sinensis*] extract, to (b) date [*Phoenix dactylifera*] product, is from 20:1 to 1:20, such as 15:1 to 1:15, such as 10:1 to 1:10, such as 5:1 to 1:5, such as 4:1 to 1:4, such as 3:1 to 1:3, such as 2:1 to 1:3, such as 1:1 to 1:3. In some embodiments the weight ratio of (a) green tea [*Camellia sinensis*] extract, to (b) date [*Phoenix dactylifera*] product, is from 20: 1 to 1:20, such as 15:1 to 1:15, such as 10:1 to 1:10, such as 5:1 to 1:5, such as 4:1 to 1:4, such as 3:1 to 1:3, such as 2:1 to 1:3, such as 1:1 to 1:3.

With regard to specific amounts of (a) green tea [*Camellia sinensis*] extract and (b) date [*Phoenix dactylifera*] product, it will be appreciated by one skilled in the art that each of the amount stated amounts of (a) green tea [*Camellia sinensis*] extract and (b) date [*Phoenix dactylifera*] product may be combined.

### *Applications*

The composition described in this specification can, in part, be an antioxidant composition. Hence, the composition may be utilised in any application in which inhibition of oxidation is required. As discussed in this specification, usage in foodstuffs is found to be particularly advantageous. In some embodiments, the present disclosure therefore provides a process for preventing and/or inhibiting oxidation of a material, comprising contacting the material with a composition comprising (a) a green tea [*Camellia sinensis*] extract; and (b) a date [*Phoenix dactylifera*] product, wherein the date product is selected from date powder, date fermentate, date extract and mixtures thereof.

In some embodiments the present disclosure also provides use of a composition comprising (a) a green tea [*Camellia sinensis*] extract; and (b) a date [*Phoenix dactylifera*] product, wherein the date product is selected from date powder, date fermentate, date extract and mixtures thereof, for preventing and/or inhibiting oxidation of a material.

The composition described in this specification can also be, in a part, a flavourant composition. Hence, the composition may also be utilised in any application in which a flavourant is required such as in foodstuffs. In some embodiments the present disclosure provides use of a composition comprising (a) a green tea [*Camellia sinensis*] extract; and (b) a date [*Phoenix dactylifera*] product, wherein the date product is selected from date powder, date fermentate,

date extract and mixtures thereof, for imparting flavour to a material such as a foodstuff.

The composition can particularly be incorporated into an emulsion. Thus there is provided an emulsion comprising a composition as described in this specification. In some embodiments, the emulsion is selected from oil-in water emulsions, water-in-oil emulsions and double emulsions. A double emulsion is an emulsion in an emulsion. In some embodiments, the double emulsion may be a water-in-oil-in-water emulsion. In some embodiments, the double emulsion may be an oil-in-water-in-oil emulsion.

The emulsion may be, for example, a foodstuff ora home and personal care product. In some embodiments, the emulsion is a foodstuff. In some embodiments, the emulsion is a home and personal care product.

Many home and personal care products may be protected by the present disclosure. Typical home and personal care products include: household cleaning products; laundry products; disinfectants; skin care products; hair care products; oral hygiene products; beauty products; cosmetics, fragrances and over the counter medicines.

In a further aspect there is provided a foodstuff comprising a composition as described in this specification.

***Foodstuff***

The composition, process and use of the present disclosure may prevent and/or inhibit oxidation in any material. However, in view of the problems associated with oxidation of foodstuffs and in view of the particular effectiveness of the present disclosure in foodstuffs, in some embodiments the composition is a foodstuff or may be added to a foodstuff. The composition and use of the present disclosure may also impart flavour to a foodstuff.

When the present composition is a foodstuff the essential components of (a) a green tea [*Camellia sinensis*] extract; and (b) a date [*Phoenix dactylifera*] product must be present in the foodstuff. They may have been provided by one or more means. For example they may have been added in the form of a composition containing the components. The components may have been added to the foodstuff sequentially.

In some embodiments, the composition of the present disclosure is an antioxidant composition suitable for addition to a foodstuff. In other embodiments, the composition of the present disclosure is a flavourant composition suitable for addition to a foodstuff.

Many foodstuffs may be protected by the present disclosure. Typical foodstuffs are raw meat, cooked meat, raw poultry products, cooked poultry products, raw seafood products, cooked seafood products, cereals, cereal bars, ready to eat meals, pasta sauces, pasteurised soups, mayonnaise, salad dressings, oil-in-water emulsions, margarines, low fat spreads, water-in-oil emulsions, dairy products, cheese spreads, processed cheese, dairy desserts, flavoured milks, cream, fermented milk products, cheese, butter, condensed milk products, ice cream mixes, soya products, pasteurised liquid egg, bakery products, confectionery products, fruit products, and foods with fat-based or water-containing fillings. In some embodiments, the food stuff is mayonnaise, salad dressings, oil-in-water emulsions and water-in-oil emulsions. In some embodiments, the foodstuff is mayonnaise. In other embodiments, the foodstuff is salad dressings. The foodstuff can also be a beverage.

In some embodiments the present composition is dosed in a foodstuff in an amount of no greater than 1 wt.% based on the weight of the foodstuff, such as no greater than 0.9 wt.%, such as no greater than 0.8 wt.%, such as no greater than 0.7 wt.%, such as no greater than 0.6 wt.%, such as no greater than 0.5 wt.%, such as no greater than 0.4 wt.%, such as no greater than 0.3 wt.%, such as no greater than 0.2 wt.%, such as no greater than 0.1 wt.%, such as no greater than 0.09 wt.%, such as no greater than 0.08 wt.%, such as no greater than 0.07 wt.%, such as no greater than 0.06 wt.%, such as no greater than 0.05 wt.%, such as no greater than 0.04 wt.%, such as no greater than 0.03 wt.%, such as no greater than 0.02 wt.%, such as no greater than 0.01 wt.%. In some embodiments, the present composition is dosed in a foodstuff in an amount of 0.01 wt.% to 1 wt.%, such as 0.01 to 0.9 wt.%, such as 0.01 to 0.8 wt.%, such as 0.01 to 0.7 wt.%, such as 0.01 to 0.6 wt.%, such as 0.01 to 0.5 wt.%, such as 0.01 to 0.4 wt.%, such as 0.01 to 0.3 wt.%, such as 0.01 to 0.2 wt.%, such as 0.01 to 0.1 wt.%, 0.02 wt.% to 1 wt.%, such as 0.02 to 0.9 wt.%, such as 0.02 to 0.8 wt.%, such as 0.02 to 0.7 wt.%, such as 0.02 to 0.6 wt.%, such as 0.02 to 0.5 wt.%, such as 0.02 to 0.4 wt.%, such as 0.02 to 0.3 wt.%, such as 0.02 to 0.2 wt.%, such as 0.02 to 0.1 wt.%, such as 0.03 wt.% to 1 wt.%, such as 0.03 to 0.9 wt.%, such as 0.03 to 0.8 wt.%, such as 0.03 to 0.7 wt.%, such as 0.03 to 0.6 wt.%, such as 0.03 to 0.5 wt.%, such as 0.03 to 0.4 wt.%, such as 0.03 to 0.3 wt.%, such as 0.03 to 0.2 wt.%, such as 0.03 to 0.1 wt.%, such as 0.04 wt.% to 1 wt.%, such as 0.04 to 0.9 wt.%, such as 0.04 to 0.8 wt.%, such as 0.04 to 0.7 wt.%, such as 0.04 to 0.6 wt.%, such as 0.04 to 0.5 wt.%, such as 0.04 to 0.4 wt.%, such as 0.04 to 0.3 wt.%, such as 0.04 to 0.2 wt.%, such as 0.04 to 0.1 wt.%, such as 0.05 wt.% to 1 wt.%, such as 0.05 to 0.9 wt.%, such as 0.05 to 0.8 wt.%, such as 0.05 to 0.7 wt.%, such as 0.05 to 0.6 wt.%, such as 0.05 to 0.5 wt.%, such as 0.05 to 0.4 wt.%, such as 0.05 to 0.3 wt.%, such as 0.05 to 0.2 wt.%, such as 0.05 to 0.1 wt.%, such as 0.06 wt.% to 1 wt.%, such as 0.06 to 0.9 wt.%, such as 0.06 to 0.8 wt.%, such as 0.06 to 0.7 wt.%, such as 0.06 to 0.6 wt.%, such as 0.06 to 0.5 wt.%, such as 0.06 to 0.4 wt.%, such as 0.06 to 0.3 wt.%, such as 0.06 to 0.2 wt.%, such as 0.06 to 0.1 wt.%, such as 0.07 wt.% to 1 wt.%, such as 0.07 to 0.9 wt.%, such as 0.07 to 0.8 wt.%, such as 0.07 to 0.7 wt.%, such as 0.07 to 0.6 wt.%, such as 0.07 to 0.5 wt.%, such as 0.07 to 0.4 wt.%, such as 0.07 to 0.3 wt.%, such as 0.07 to 0.2 wt.%, such as 0.07 to 0.1 wt.%, such as 0.08 wt.%

to 1 wt.%, such as 0.08 to 0.9 wt.%, such as 0.08 to 0.8 wt.%, such as 0.08 to 0.7 wt.%, such as 0.08 to 0.6 wt.%, such as 0.08 to 0.5 wt.%, such as 0.08 to 0.4 wt.%, such as 0.08 to 0.3 wt.%, such as 0.08 to 0.2 wt.%, such as 0.08 to 0.1 wt.%, such as 0.09 wt.% to 1 wt.%, such as 0.09 to 0.9 wt.%, such as 0.09 to 0.8 wt.%, such as 0.09 to 0.7 wt.%, such as 0.09 to 0.6 wt.%, such as 0.09 to 0.5 wt.%, such as 0.09 to 0.4 wt.%, such as 0.09 to 0.3 wt.%, such as 0.09 to 0.2 wt.%, such as 0.09 to 0.1 wt.%.

In some embodiments, the present composition is dosed in a foodstuff in an amount to provide the green tea [*Camellia sinensis*] extract in an amount of no greater than 0.5 wt.% based on the weight of the foodstuff, such as no greater than 0.4 wt.%, such as no greater than 0.3 wt.%, such as no greater than 0.2 wt.%, such as no greater than 0.1 wt.%, such as no greater than 0.07 wt.%, such as no greater than 0.05 wt.%, such as no greater than 0.04 wt.%, such as no greater than 0.03 wt.%, such as no greater than 0.02 wt.%, such as no greater than 0.025 wt.%, such as no greater than 0.015 wt.%, such as no greater than 0.01 wt.%, such as no greater than 0.0075 wt.%, such as no greater than 0.005 wt.%, such as from 0.5 to 1 wt.%, such as from 0.4 to 1 wt.%, such as from 0.3 to 1 wt.%, such as from 0.2 to 1 wt.%, such as from 0.01 to 1 wt.%, such as from 0.07 to 1 wt.%, such as from 0.05 to 1 wt.%, such as from 0.04 to 1 wt.%, such as from 0.03 to 1 wt.% such as from 0.02 to 1 wt.% based on the weight of the foodstuff.

In some embodiments, the present composition is dosed in a foodstuff in an amount to provide the date [*Phoenix dactylifera*] product in an amount of no greater than 1 wt.% based on the weight of the foodstuff, such as no greater than 0.8 wt.%, such as no greater than 0.6 wt.%, such as no greater than 0.4 wt.%, such as no greater than 0.2 wt.%, such as no greater than 0.08 wt.%, such as no greater than 0.05 wt.%, such as no greater than 0.04 wt.%, such as no greater than 0.03 wt.% such as no greater than 0.02 wt.%, such as no greater than 0.025 wt.% such as no greater than 0.015 wt.%, such as no greater than 0.01 wt.%, such as no greater than 0.0075 wt.%, such as no greater than 0.005 to 1 wt.%, such as from 0.5 to 1 wt.%, such as from 0.4 to 1 wt.%, such as from 0.3 to 1 wt.%, such as from 0.2 to 1 wt.%, such as from 0.01 to 1 wt.%, such as from 0.07 to 1 wt.%, such as from 0.05 to 1 wt.%, such as from 0.04 to 1 wt.%, such as from 0.03 to 1 wt.% such as from 0.02 to 1 wt.% based on the weight of the foodstuff.

### *Additional Components*

The composition of the present disclosure or the composition for use in the present disclosure may contain one or more additional components. However, in some embodiments the composition of the present disclosure (suitable for addition to a foodstuff) contains no additional components or contains no additional components that materially affect the properties of the composition.

In these embodiments, the present disclosure provides a composition consisting essentially of or consisting of (a) a green tea [*Camellia sinensis*] extract; and (b) a date [*Phoenix dactylifera*] product, wherein the date product is selected from date powder, date fermentate, date extract and mixtures thereof.

In other embodiments, the present disclosure provides a process for preventing and/or inhibiting oxidation of an emulsion or a foodstuff, the process comprising the steps of contacting the emulsion or foodstuff with a composition consisting essentially of or consisting of (a) a green tea [*Camellia sinensis*] extract; and (b) a date [*Phoenix dactylifera*] product, wherein the date product is selected from date powder, date fermentate, date extract and mixtures thereof.

In other embodiments, the present disclose provides use of a composition consisting essentially of or consisting of (a) a green tea [*Camellia sinensis*] extract; and (b) a date [*Phoenix dactylifera*] product, wherein the date product is selected from date powder, date fermentate, date extract and mixtures thereof, for preventing and/or inhibition oxidation of an emulsion or a foodstuff.

In other embodiments, the present disclose provides use of a composition consisting essentially of or consisting of (a) a green tea [*Camellia sinensis*] extract; and (b) a date [*Phoenix dactylifera*] product, wherein the date product is selected from date powder, date fermentate, date extract and mixtures thereof, for imparting flavour to an emulsion or a foodstuff.

In other embodiments the composition further comprises a carrier. In come embodiments the carrier is selected from propylene glycol, maltodextrin, sugar, salt, ethanol, water, protein, glycerol, medium chain triglyceride (MCT oil), and vegetable oil. The components of the present disclosure, that is (a) a green tea [*Camellia sinensis*] extract; and (b) a date [*Phoenix dactylifera*] product, wherein the date product is selected from date powder, date fermentate, date extract and mixtures thereof, may be blended with the carrier or carriers in any suitable manner. For example, the components of the present disclosure may be simply mixed with a suitable carrier or carriers.

In some embodiments, the composition comprises a carrier (such as maltodextrin) in an amount of from 1 to 40 wt.% based on the composition. In some embodiments, the composition comprises a carrier (such as maltodextrin) in an amount of from 2 to 35 wt.% based on the composition. In some embodiments, the composition comprises a carrier (such as maltodextrin) in an amount of from 5 to 30 wt.% based on the composition. In some embodiments, the composition comprises a carrier (such as maltodextrin) in an amount of from 5 to 25 wt.% based on the composition. In some embodiments, the composition comprises a carrier (such as maltodextrin) in an amount of from 10 to 30 wt.% based on the composition. In some embodiments, the composition comprises a carrier (such as maltodextrin) in an amount of

from 10 to 25 wt.% based on the composition. In some embodiments, the composition comprises a carrier (such as maltodextrin) in an amount of from 15 to 25 wt.% based on the composition.

In some embodiments the composition further comprises an emulsifier. In some embodiments the emulsifier is selected from polyoxyethylene sorbitan esters (polysorbates), polyoxyethylene stearate, mono- and diglycerides of fatty acids, mono- and diglycerides esters further esterified with a dibasic organic acid selected from acetic acid, lactic acid, citric acid and mono- and diacetyl tartaric acid or mixtures thereof, lecithin, polyglycerol esters of fatty acids, polyglycerol polyricinoleate, sucrose esters of fatty acids, sucroglycerides, propylene glycol esters of fatty acids, sorbitan esters of fatty acids, sodium and calcium salt of stearoyl-2-lactylate, sodium, potassium, calcium and magnesium salts of fatty acids and ammonium phosphatides.

### *Process*

As discussed in this specification, the present disclosure provides a process for preventing and/or inhibiting oxidation of a foodstuff or an emulsion the process comprising the step of contacting the foodstuff or the emulsion with a composition comprising (a) a green tea [*Camellia sinensis*] extract; and (b) a date [*Phoenix dactylifera*] product, wherein the date product is selected from date powder, date fermentate, date extract and mixtures thereof.

In some embodiments, the green tea [*Camellia sinensis*] extract and the date [*Phoenix dactylifera*] product are added to the foodstuff or emulsion together. In other embodiments, the the green tea [*Camellia sinensis*] extract and the date [*Phoenix dactylifera*] product are added to the foodstuff or the emulsion sequentially.

Thus the present disclosure provides in some embodiments an antioxidant composition which may be added to a range of materials such as food systems and in other embodiments a combination of two separate products which may added sequentially to materials such as food products.

The present disclosure will now be described in further detail by way of example only with reference to the accompanying figures in which:

FIG. **1A - 1D** show a graph;
FIG. **2A - 2B** show a graph; and
FIG. **3A - 3D** shows a graph.

The present disclosure will now be described in further detail in the following examples.

## EXAMPLES

Three individual trials were conducted in respect of the synergistic interaction between green tea extract and a date product in a foodstuff. Determination of peroxide concentration (PV) by ferric thiocyanate method and malondialdehyde (MDA) concentration by TBARS assay revealed a synergistic interaction between green tea extract (GTE) and both date powder (DP) and date fermentate (DF). As illustrated below, when green tea extract is combined with date powder or date fermentate the combination exhibits an antioxidant effect that is greater than a simple additive effect.

### Materials used

**Green tea extract** (Guardian® Green Tea Extract 20M by DuPont) was an extract from green tea leaves and contained approximately 20% of catechins.

**Date Powder** was a powder prepared from pitted, diced and dried date through hammer milling.

**Date fermentate** was prepared according to the procedure set out in US5096718. Date powder (prepared from pitted date by a dicing-drying-milling process) was used as the source of carbohydrate for fermentation. In particular Example 3 of US5096718 was repeated using the date powder as a source of carbohydrate for fermentation in place of milk. The liquid fermentate obtained is spray dried to obtain a powder fermentate.

### Methods

### *Determination of peroxide value*

Peroxide value was determined by ferric thiocyanate method according to IDF (International Dairy Federation) Standards 74A: 1991 with modifications. Briefly, 1.5 ml of isooctane/isopropanol (3/2) was added to 0.3g of ranch dressing or mayonnaise sample and the content mixed by shaking and vortexing. The mixture was then centrifuged at 3500 ppm for 5 minutes, and an aliquot (0.2 ml) of the supernatant was transferred to another tube, to which 2.8 ml of methanol/butanol (2/1) and 30 μl of $Fe^{2+}$/thiocyanate reagent were added. The absorbance of the mixture was measured after exactly

20 minutes at 510 nm. $Fe^{2+}$/thiocyanate reagent was prepared by mixing equal volumes of ammonium thiocyanate (3.94M) and $Fe^{2+}$ solution (freshly prepared by mixing 0.132M barium chloride and 0.144M ferrous sulfate at 1:1 followed by centrifugation). Peroxide value was calculated from a standard curve of cumene hydroperoxide.

***Determination of MDA value by TBARS assay***

Secondary oxidation products (TBARS as MDA equivalent) were measured by a spectrophotometric method on the oil separated from the ranch dressing or mayonnaise samples by freeze-thaw-centrifuge treatment. Briefly, 0.25 g of the oil was added to 3 ml of TBA reagent (0.25% thiobarbituric acid, 10% trichloroacetic acid, 0.16M HCl and 0.02% pyrogallol). The mixture was heated in a water bath at 100°C for 15 minutes and centrifuged at 12,000g for 5 minutes after cooling down to room temperature. The absorbance of the clear solution was measured at 532nm, and results were calculated from a standard curve of tetraethoxypropane.

***Determination of % inhibition***

The inhibition percentages of the treatments were calculated relative to the untreated control using the following equation:

$$((C_{control} - C_{measurement})/\ (C_{control}) * 100\%$$

where

$C_{control}$= concentration of peroxide or MDA in the control
$C_{measurment}$ = concentration of peroxide or MDA in the treatment.

**1. Demonstration of green tea extract (GTE) and date powder (DP) synergy in mayonnaise**

In the present example a mixture of green tea [*Camellia sinensis*] extract (GTE) and date powder (DP) was investigated in mayonnaise.

The mayonnaise was produced according to the formulation in Table 1 and the procedure outlined below. All ingredients in the mayonnaise were of food-grade quality. The dosage was based on the total product mayonnaise weight. The dosage of green tea extract (GTE) was 100 ppm. The dosage of date powder (DP) was 400 ppm.

**Table 1. Mayonnaise formulation (%)**

| Ingredient | Mayonnaise with no antioxidants (CTR) | Mayonnaise with 100 ppm green tea extract (GTE) | Mayonnaise with 400 ppm date powder extract (DP) | Mayonnaise with 100 ppm green tea extract and 400ppm date powder (GTE + DP) |
|---|---|---|---|---|
| Soybean Oil | 70 | 70 | 70 | 70 |
| Salt | 1 | 1 | 1 | 1 |
| Sucrose | 1.6 | 1.6 | 1.6 | 1.6 |
| Vinegar | 4 | 4 | 4 | 4 |
| Mustard powder | 0.25 | 0.25 | 0.25 | 0.25 |
| Egg Yolk Powder | 3 | 3 | 3 | 3 |
| Potassium Sorbate (20% solution) | 0.5 | 0.5 | 0.5 | 0.5 |
| Green tea extract | ---- | 0.01 | ---- | 0.01 |

(continued)

| Ingredient | Mayonnaise with no antioxidants (CTR) | Mayonnaise with 100 ppm green tea extract (GTE) | Mayonnaise with 400 ppm date powder extract (DP) | Mayonnaise with 100 ppm green tea extract and 400ppm date powder (GTE + DP) |
|---|---|---|---|---|
| Date powder | ---- | ---- | 0.04 | 0.04 |
| Water | 19.65 | 19.64 | 19.61 | 19.60 |
| **TOTAL** | 100 | 100 | 100 | 100 |

***Procedure:*** Water and sorbate were added to Thermomix with butterfly attachment followed by addition of sugar and antioxidant. The content was mixed at speed 2 for 2 minutes. The remaining ingredients except oil and acids were added and mixed for 1 min at speed 3. Oil was added in slowly and mixed at speed 3.5 for 1.5 minutes followed by addition of vinegar. The content was emulsified under high shear at speed 6 for 2 minutes, and pH was adjusted to 3.8-4.0 with lactic acid.

***Results***

The mayonnaise was stored at 35 °C. A sample of the mayonnaise was analysed after 20 days and the concentration of peroxide and MDA was measured. A second sample of the mayonnaise was analysed after 44 days and the concentration of peroxide and MDA was measured. The experiments were conducted in duplicate and the average values of peroxide (measured as meq/kg of mayonnaise) and MDA (measured as μmol MDA eq./kg of mayonnaise) are presented in Table 2 below and graphically viewed in Figures 1A - 1D.

**Table 2. Peroxide (meq/kg) and MDA (μ mol MDA eq./Kg) values of mayonnaise after 20 and 44 days of storage at 35°C.**

| Treatment | 20 days | | | | 44 days | | | |
|---|---|---|---|---|---|---|---|---|
| | PV | % inhibition | MDA | % inhibition | PV | % inhibition | MDA | % inhibition |
| control | 16.13 | 0.00 | 13.69 | 0.00 | 33.72 | 0.00 | 25.94 | 0.00 |
| GTE | 14.33 | 11.16 | 14.53 | -6.18 | 31.34 | 7.06 | 29.96 | -15.52 |
| DP | 17.70 | -9.72 | 18.52 | -35.30 | 33.34 | 1.15 | 24.79 | 4.43 |
| GTE + DP | 14.32 | 11.24 | 14.06 | -2.75 | 29.22 | 13.35 | 20.95 | 19.23 |

As can be seen above, the date powder exhibited a pro-oxidant effect after 20 days in both experiments. In contrast, after 44 days the date powder exhibited an antioxidant effect in both experiments. Without being bound in this regard, this is thought to be related to the localization/migration and concentration change of the date powder components in different phases and interface of the emulsion system which may be concurrent with the emulsion phase change during the storage of mayonnaise.

It is noted that after 20 and 44 days the green tea extract exhibited an antioxidant effect in the peroxide experiments, whereas, it exhibited a pro-oxidant effect in the TBARS experiment. Without being bound in this regard, this is thought to be a consequence of the peroxides breaking down into secondary oxidation products such as TBARS. At a certain stage of oxidation, the rate of peroxides breakdown may outweigh their rate of formation; therefore, a descending trend may be observed.

Based on the above values measured for each individual component, one skilled in the art would have expected the combination of green tea extract and date powder to exhibit a greater pro-oxidant effect in the TBARS assay after 20 days. However, this was not the case and the combination of green tea extract and date powder exhibited a reduced pro-oxidant effect in the TBARS assay. Moreover, in the peroxide experiment, the combination of green tea extract and date powder exhibited a peroxides reduction effect that was greater than the sum of the two individual components.

At 44 days, both the peroxide and TBARS experiment demonstrate that the combination of green tea extract and date powder has a greater antioxidant effect than the sum of the individual components. Thus, the above experiment illustrates the synergistic interaction between green tea extract and date powder.

**2. Demonstration of green tea extract (GTE) and date powder (DP) synergy in ranch dressing**

In the present example a two-component mixture of green tea [*Camellia sinensis*] extract (GTE) and date powder (DP) was investigated in ranch dressing.

The ranch dressing was produced according to the formulation in Table 3 and the procedure outlined below. All ingredients were of food-grade quality. The dosage was based on the total product ranch dressing weight. The dosage of green tea extract (GTE) was 250 ppm. The dosage of date powder (DP) was 10,000 ppm.

**Table 3. Ranch dressing formulation (%).**

| **Ingredients** | **Ranch dressing with no antioxidants (CTR)** | **Ranch dressing with 250 ppm green tea extract (GTE)** | **Ranch dressing with 10,000 ppm date powder extract (DP)** | **Ranch dressing with 250 ppm green tea extract and 10,000ppm date powder (GTE + DP)** |
|---|---|---|---|---|
| Water | 50.6 | 50.6 | 49.6 | 49.6 |
| Potassium sorbate, 20% solution | 0.5 | 0.5 | 0.5 | 0.5 |
| Stabilizer (guar and xanthan gum) | 0.3 | 0.3 | 0.3 | 0.3 |
| Sucrose | 1 | 1 | 1 | 1 |
| Egg Yolk Powder | 2.5 | 2.5 | 2.5 | 2.5 |
| Cultured Buttermilk | 1 | 1 | 1 | 1 |
| Soybean Oil | 40 | 40 | 40 | 40 |
| Salt | 1 | 1 | 1 | 1 |
| Cider Vinegar | 3 | 3 | 3 | 3 |
| Lactic Acid, 88% solution | 0.1 | 0.1 | 0.1 | 0.1 |
| Green tea extract | ---- | 0.025 | ---- | 0.025 |
| Date Powder | ---- | ---- | 1 | 1 |
| **TOTAL** | 100 | 100 | 100 | 100 |

***Procedure:*** Sorbate and water were added to the Thermomix, followed by addition of the sugar/stabilizer/antioxidant blend. The content was mixed at speed 4 for 5 minutes. Egg yolk powder and buttermilk were added and mixed for 2 minutes at speed 4. Soybean oil was added slowly and mixed at speed 5 for 1 minute. Next, salt was added and mixed for 2 minutes at speed 6. Acid and vinegar were added last and mixed for 1 minute at speed 3.5. The content was emulsified under high shear for 2 minutes, and pH adjusted to 4.0-4.2 with lactic acid.

***Results***

The ranch dressing was stored at 35 °C. A sample of the ranch dressing was analysed after 14 days and the concentration of MDA was measured. A second sample of the ranch dressing was analysed after 35 days of storage and the concentration of MDA was measured. The experiments were conducted in duplicate and the average values of MDA (measured as μmol MDA eq./kg of ranch dressing) are presented in Table 4 below and graphically viewed in Figures 2A - 2B.

**Table 4. TBARS (μ mol MDA eq./Kg) of ranch dressing after 14 and 35 days of storage at 35°C.**

| Treatment | 14 days | | 35 days | |
|---|---|---|---|---|
| | MDA | % inhibition | MDA | % inhibition |
| **Control** | 6.89 | 0.00 | 9.36 | 0.00 |
| **GTE** | 4.54 | 34.11 | 9.42 | -0.63 |
| **DP** | 5.93 | 13.90 | 10.83 | -15.75 |
| **GTE + DP** | 2.84 | 58.82 | 8.07 | 13.73 |

After 14 days both green tea extract and date powder exhibited an antioxidant effect. This antioxidant effect was increased when the components were combined. Moreover, the improved antioxidant effect was greater than the sum of the two components alone, thus, demonstrating a synergistic interaction between the green tea extract and the date powder.

It is noted that after 35 days both green tea extract and date powder alone exhibited a pro-oxidant effect. As explained above, this is thought to be a consequence of the antioxidant localization and concentration change in the multi-phase emulsion system of ranch dressing. Nonetheless, the combination of green tea extract and date powder at 35 days reversed this effect and demonstrated an antioxidant effect. This could not have been predicted from the measured values of each individual component and demonstrates the synergistic interaction between green tea extract and date powder.

**3. Demonstration of green tea extract (GTE) and date fermentate (DF) synergy in ranch dressing**

In the present example a two-component mixture of green tea extract (GTE) and date fermentate (DF) was investigated in ranch dressing.

The ranch dressing was produced according to the formulation in Table 5 and the procedure outlined below. All ingredients were of food-grade quality. The dosage was based on the total product ranch dressing weight. The dosage of green tea extract (GTE) was 300 ppm. The dosage of date fermentate (DF) was 10,000 ppm.

**Table 5. Ranch dressing formulation (%).**

| Ingredients | Ranch dressing with no antioxidants (CTR) | Ranch dressing with 300 ppm green tea extract (GTE) | Ranch dressing with 10,000 ppm date fermentate extract (DF) | Ranch dressing with 300 ppm green tea extract and 10,000ppm date fermentate (GTE + DP) |
|---|---|---|---|---|
| Soybean Oil | 40 | 40 | 40 | 40 |
| Egg Yolk Powder | 2.5 | 2.5 | 2.5 | 2.5 |
| Sugar | 1 | 1 | 1 | 1 |
| Stabilizer (guar and xanthan gum) | 0.35 | 0.35 | 0.35 | 0.35 |
| Cider Vinegar | 3 | 3 | 3 | 3 |
| Salt | 1 | 1 | 1 | 1 |
| Buttermilk | 1 | 1 | 1 | 1 |
| Lactic Acid, 90% | 0.1 | 0.1 | 0.1 | 0.1 |
| Potassium Sorbate, 20% | 0.5 | 0.5 | 0.5 | 0.5 |

(continued)

| Ingredients | Ranch dressing with no antioxidants (CTR) | Ranch dressing with 300 ppm green tea extract (GTE) | Ranch dressing with 10,000 ppm date fermentate extract (DF) | Ranch dressing with 300 ppm green tea extract and 10,000ppm date fermentate (GTE + DP) |
|---|---|---|---|---|
| Water | 50.55 | 50.52 | 49.55 | 49.47 |
| Green tea extract | ---- | 0.03 | ---- | 0.03 |
| Date fermentate | ---- | ---- | 1 | 1 |
| Total | 100 | 100 | 100 | 100 |

***Procedure:*** Sorbate and water were added to the Thermomix, followed by addition of the sugar/stabilizer/antioxidant blend. The content was mixed at speed 4 for 5 minutes. Egg yolk powder and buttermilk were added and mixed for 2 minutes at speed 4. Soybean oil was added slowly and mixed at speed 5 for 1 minute. Next, salt was added and mixed for 2 minutes at speed 6. Acid and vinegar were added last and mixed for 1 minute at speed 3.5. The content was emulsified under high shear for 2 minutes, and pH adjusted to 4.0-4.2 with lactic acid.

***Results***

The ranch dressing was stored at 30 °C. A sample of the ranch dressing was analysed after 21 days and the concentration of peroxide was measured. A second sample of the ranch dressing was analysed after 28 days of storage, a third after 35 days of storage and a fourth after 42 days of storage, and the concentration of peroxide was measured for each sample. The experiments were conducted in duplicate and the average values of peroxide (measured as meq/kg of ranch dressing) are presented in Table 6 below and graphically viewed in Figures 3A - 3D.

**Table 6. Peroxide value (meq/kg) of ranch dressing after storage at 30°C.**

| Treatment | 21 days | | 28 days | | 35 days | | 42 days | |
|---|---|---|---|---|---|---|---|---|
| | PV | % inhibition | PV | % inhibition | PV | % inhibition | PV | % inhibition |
| **Control** | 8.21 | 0.00 | 11.09 | 0.00 | 14.90 | 0.00 | 16.04 | 0.00 |
| **GTE** | 7.42 | 9.57 | 10.02 | 9.63 | 14.99 | -0.62 | 16.55 | -3.20 |
| **DF** | 6.98 | 15.02 | 8.79 | 20.73 | 12.32 | 17.34 | 13.19 | 17.80 |
| **GTE + DF** | 5.80 | 29.39 | 6.19 | 44.18 | 7.18 | 51.78 | 8.07 | 49.71 |

After 21 days and 28 days both green tea extract and date fermentate exhibit an antioxidant effect. This effect is improved when the components are combined. The improvement is far greater than the sum of the individual components alone, which demonstrates a synergistic interaction between green tea extract and date fermentate.

It is noted that the antioxidant effect of green tea extract diminishes over time. In fact, as shown above, after 35 and 42 days of storage green tea extract exhibits a pro-oxidant effect. However, when green tea extract is combined with date fermentate this effect is reversed and the combination shows an antioxidant effect. This further demonstrates the synergistic interaction between green tea extract and date fermentate.

**Example of a blend composition**

Table 7 shows an example of a blend composition of green tea extract and date powder in addition to a carrier (maltodextrin).

**Table 7: Blend composition**

| Ingredient | g/kg | % |
|---|---|---|
| Green tea extract | 47 | 4.7 |

(continued)

| Ingredient | g/kg | % |
|---|---|---|
| Date powder | 753 | 75.3 |
| Maltodextrin | 200 | 20 |
| Total | 1000 | 100 |

The blend is used in mayonnaise or ranch dressing in amounts of 50-2500 ppm based on the amount of mayonnaise or ranch dressing.

**CONCLUSION**

The studies described in this specification demonstrate that the combination of a green tea extract with a date product was more effective than the individual components in food models.

Moreover, a significant synergistic interaction between green tea extract and date product was demonstrated. The fact that green tea extract and date product interact synergistically is novel and provides the following range of functional and economic benefits compared to present commercial natural solutions: high antioxidant activity (increased product shelf life and consumer satisfaction), low cost-in-use, low impact on taste and low discolouration.

All publications mentioned in the above specification are incorporated into the specification by reference. Various modifications and variations of the described methods and system of the specification will be apparent to those skilled in the art without departing from the scope and spirit of the disclosure. Although the disclosure has been described in connection with specific embodiments, it should be understood that the disclosure as claimed should not be unduly limited to such specific embodiments. Indeed, various modifications of the described modes for carrying out the disclosure which are obvious to those skilled in chemistry, biology, food science or related fields are intended to be within the scope of the following claims

**REFERENCES**

Kubo I., Muroi H. and Himejima M., "Antimicrobial activity of green tea flavour components and their combination effects", Journal of Agricultural and Food Chemistry, 1992, 40, 245-248.

Yam T. S., Shah S. and Hamilton-Miller J. M., "Microbiological activity of whole and fractionated crude extracts of tea (Camellia sinensis), and of tea components". FEMS Microbiol. Lett., 1997, 152, 169-174.

Kajiya K., Kumazawa S., Nakayama T., "Steric effects on interaction of tea catechins with lipid bilayers", Bioscience, Biotechnology and Biochemistry, 2001, 65, 2638-2643.

Ghnimi S., Umer S., Karim A. and Kamal-Eldin A., "Date fruit (Phoenix dactylifera L.): An underutilized food seeking industrial valorization", NFS Journal, 2017, 6, 1 -10.

IDF (International Dairy Federation) Standard Methods, 1991, Sec.74A, Square Vergote 41, Brussels, Belgium.

**Claims**

1. A composition comprising:

   (a) a green tea [*Camellia sinensis*] extract; and
   (b) a date [*Phoenix dactylifera*] product,

   wherein the date product is selected from date powder, date fermentate, date extract and mixtures thereof.

2. A composition according to claim 1, wherein the date product comprises date powder, date fermentate or date extract.

**3.** A composition according to any one of the preceding claims, further comprising maltodextrin.

**4.** A composition according to any one of the preceding claims, wherein the green tea [*Camellia sinensis*] extract is present in an amount of no greater than 5 wt.% based on the total weight of the composition.

**5.** A composition according to any one of the preceding claims, wherein the green tea [*Camellia sinensis*] extract is present in an amount of at least 0.5 wt.% based on the total weight of the composition.

**6.** A composition according to any one of the preceding claims, wherein the date product is present in an amount of no greater than 80 wt.% based on the total weight of the composition.

**7.** A composition according to any one of the preceding claims, wherein the date product is present in an amount of at least 40 wt.% based on the total weight of the composition.

**8.** A composition according to any one of the preceding claims, wherein the date product is present in an amount of from 50 to 70 wt.% based on the total weight of the composition.

**9.** An emulsion comprising a composition as defined in any one of the preceding claims.

**10.** An emulsion according to claim 9, wherein the composition is present in an amount of from 0.01 to 1 wt.% based on the total weight of the emulsion.

**11.** An emulsion according to claim 9 or claim 10, wherein the emulsion is selected from oil-in water emulsions, water-in-oil emulsions and double emulsions.

**12.** A foodstuff comprising a composition as defined in any one of claims 1 to 8.

**13.** A foodstuff according to claim 12, wherein the composition is present in an amount of from 0.01 to 1 wt.% based on the total weight of the foodstuff.

**14.** A foodstuff according to claim 12 or claim 13, wherein the foodstuff is selected from mayonnaise, salad dressings, oil-in-water emulsions and water-in-oil emulsions.

**15.** A process for preventing or inhibiting oxidation of a foodstuff or an emulsion, comprising contacting a foodstuff or an emulsion with a composition as defined in any one of claims 1 to 8.

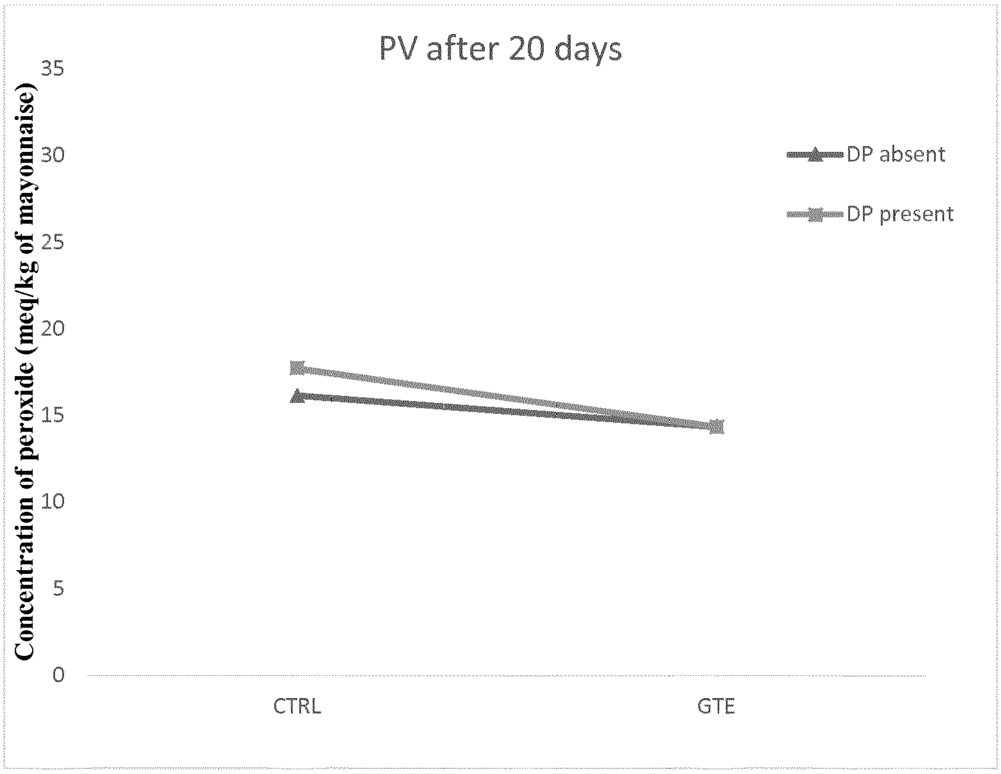
PV after 20 days
Concentration of peroxide (meq/kg of mayonnaise)
35
30
25
20
15
10
5
0
CTRL
GTE
DP absent
DP present

FIG. 1A

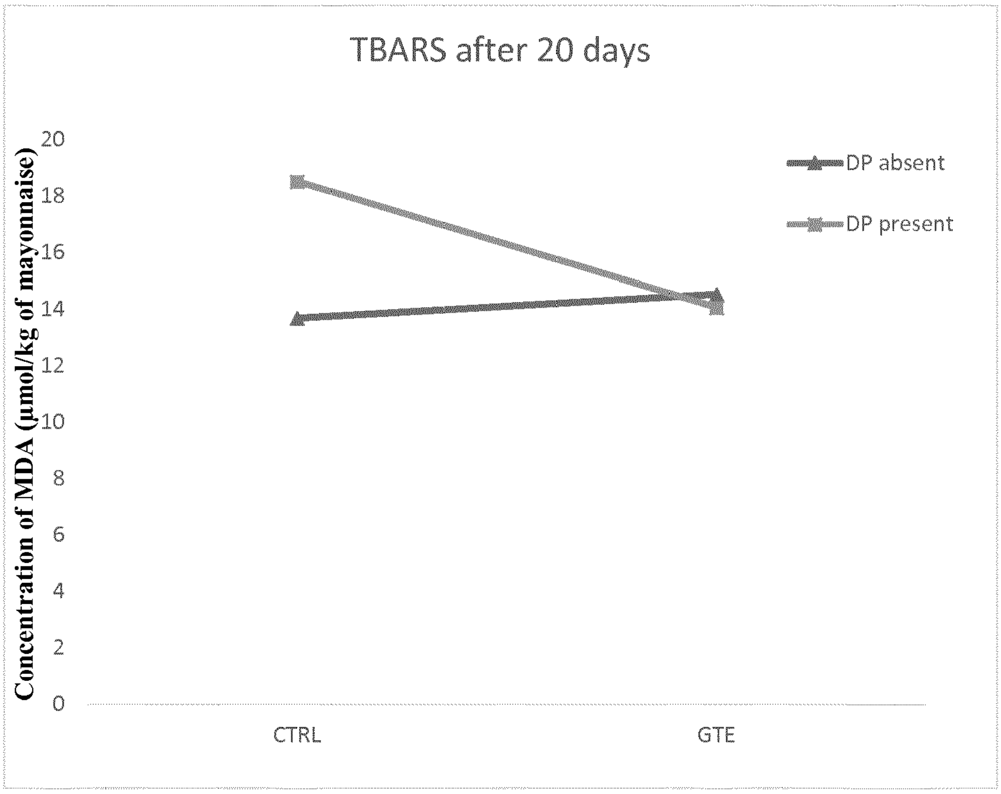
TBARS after 20 days
Concentration of MDA (µmol/kg of mayonnaise)
20
18
16
14
12
10
8
6
4
2
0
CTRL
GTE
DP absent
DP present

FIG. 1B

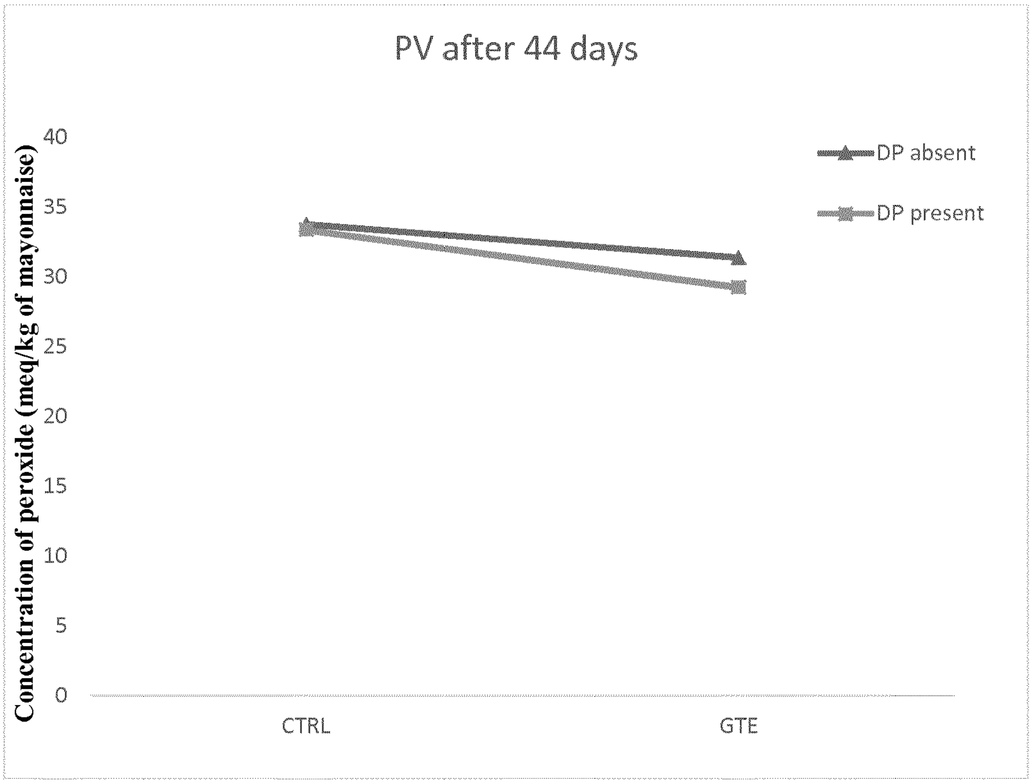
PV after 44 days
Concentration of peroxide (meq/kg of mayonnaise)
40
35
30
25
20
15
10
5
0
CTRL
GTE
DP absent
DP present

FIG. 1C

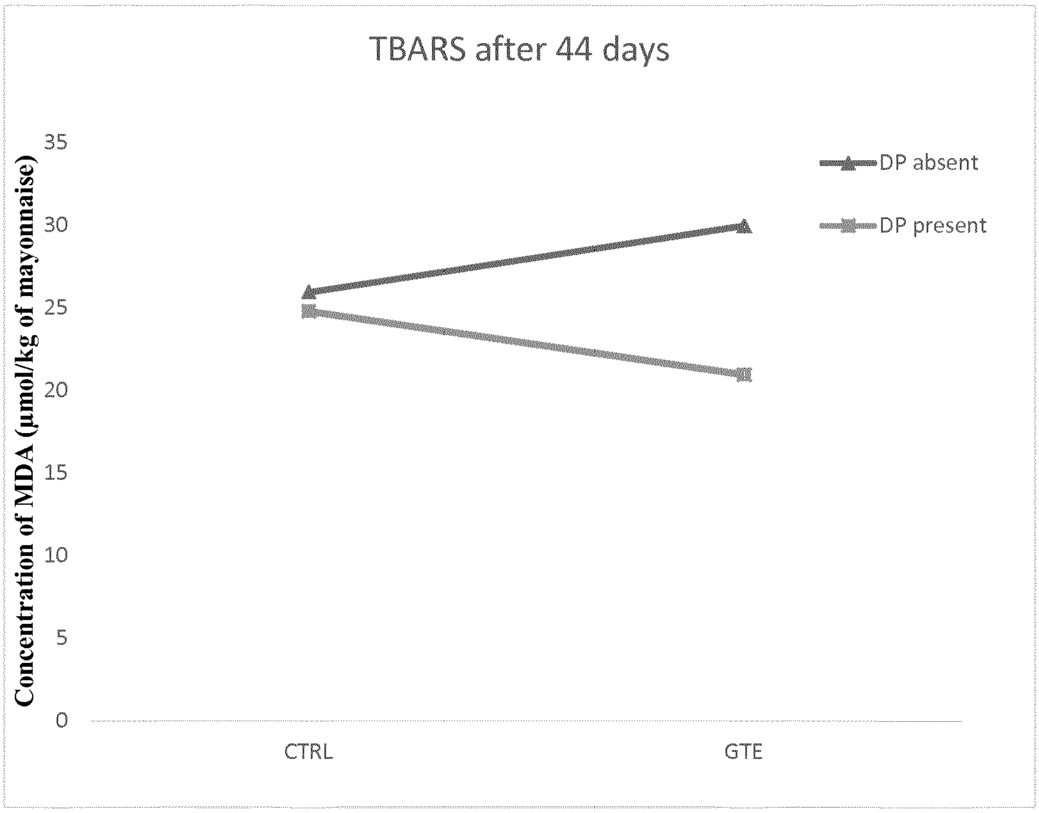
TBARS after 44 days
Concentration of MDA (µmol/kg of mayonnaise)
35
30
25
20
15
10
5
0
CTRL
GTE
DP absent
DP present

FIG. 1D

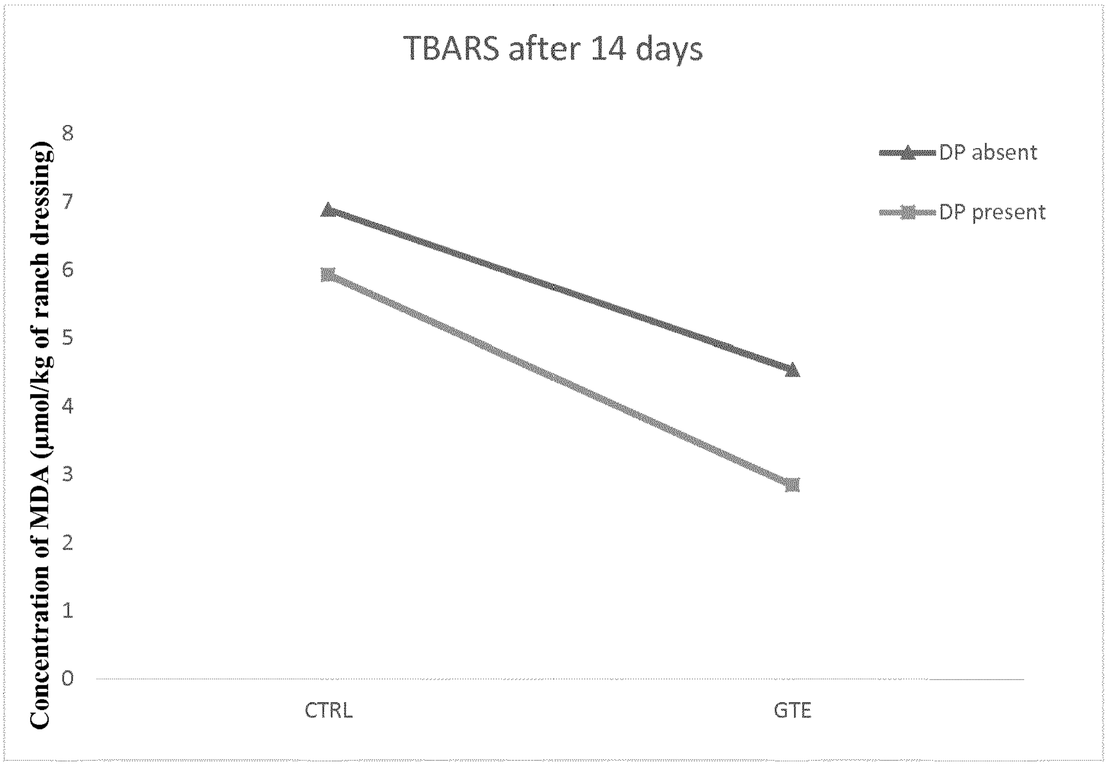

TBARS after 14 days
Concentration of MDA (µmol/kg of ranch dressing)
0
1
2
3
4
5
6
7
8
CTRL
GTE
DP absent
DP present


FIG. 2A

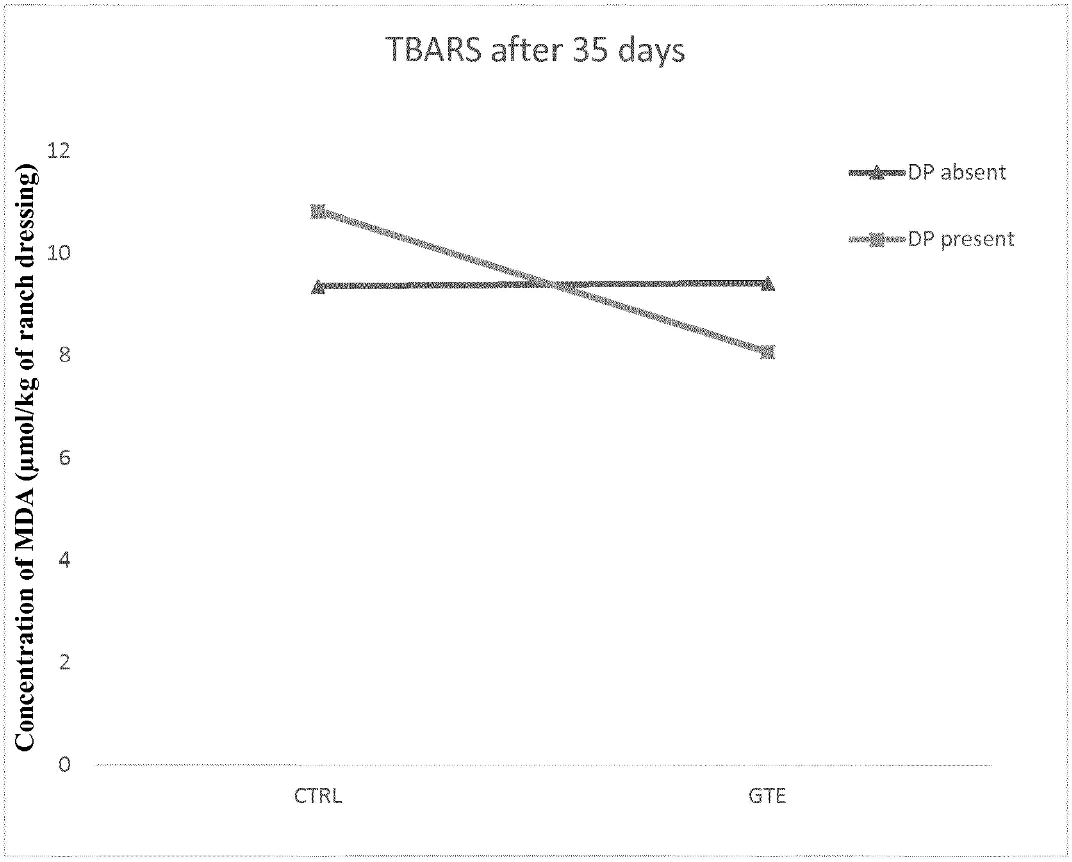

TBARS after 35 days
Concentration of MDA (µmol/kg of ranch dressing)
0
2
4
6
8
10
12
CTRL
GTE
DP absent
DP present


FIG. 2B

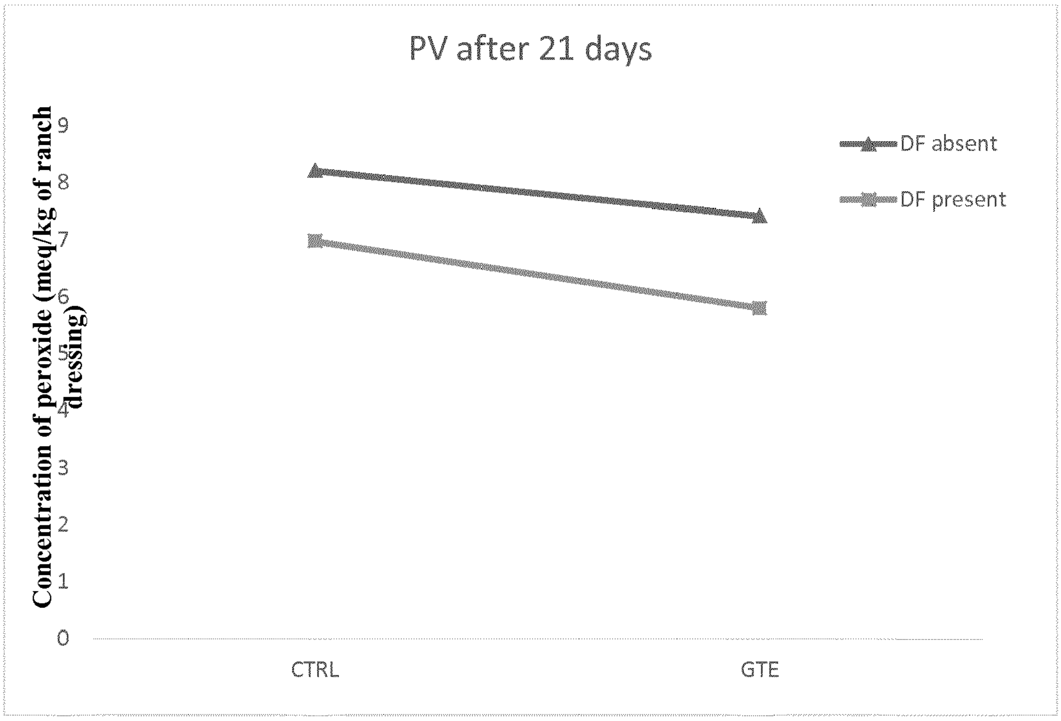
PV after 21 days
Concentration of peroxide (meq/kg of ranch dressing)
9
8
7
6
5
4
3
2
1
0
DF absent
DF present
CTRL
GTE

FIG. 3A

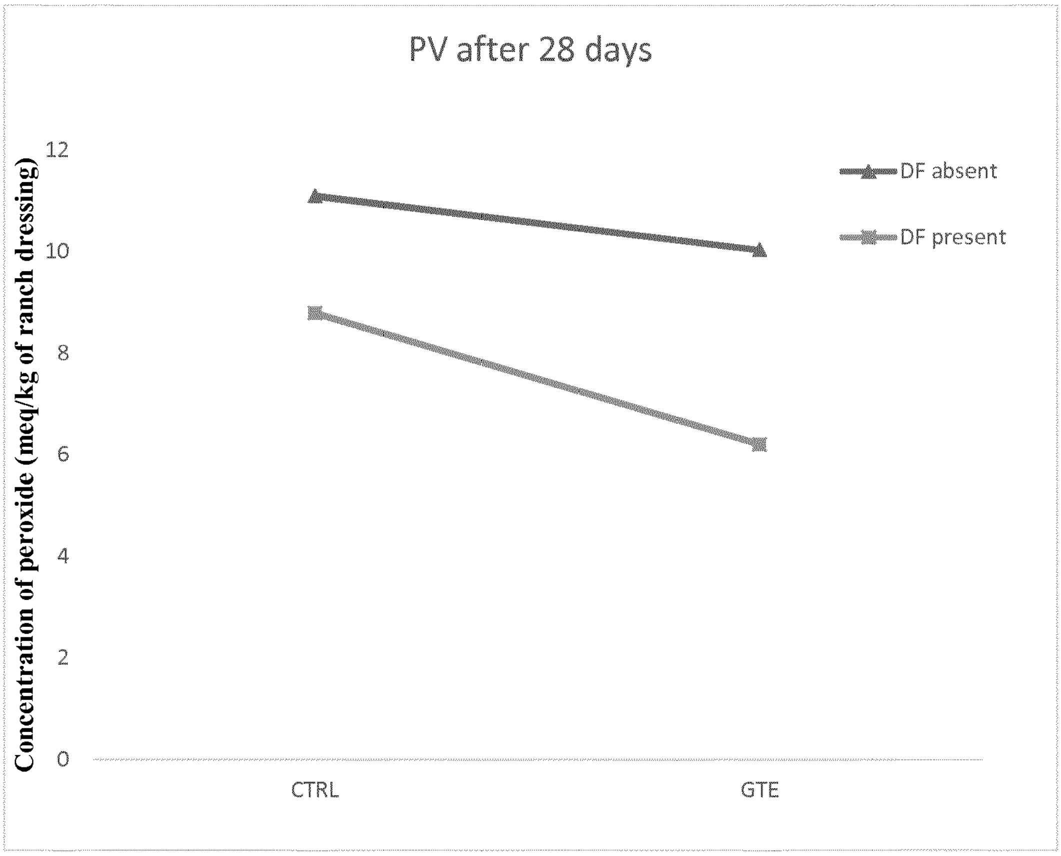
PV after 28 days
Concentration of peroxide (meq/kg of ranch dressing)
12
10
8
6
4
2
0
DF absent
DF present
CTRL
GTE

FIG. 3B

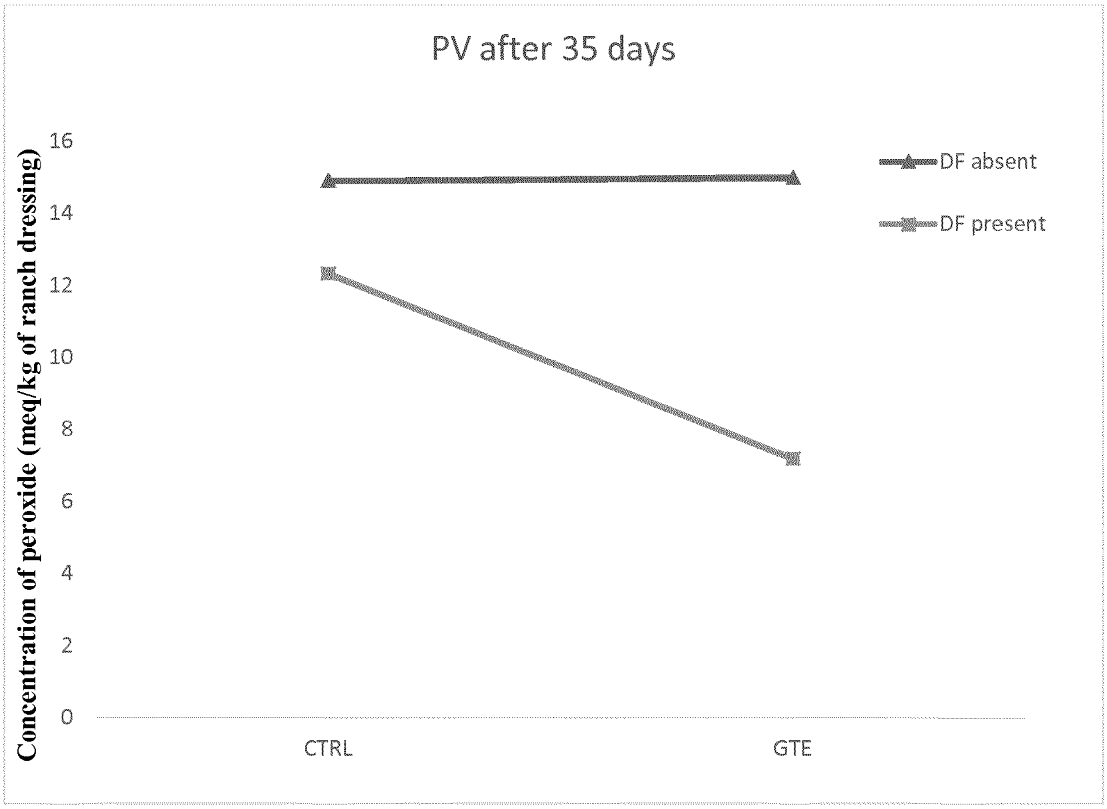
PV after 35 days
Concentration of peroxide (meq/kg of ranch dressing)
16
14
12
10
8
6
4
2
0
CTRL
GTE
DF absent
DF present

FIG. 3C

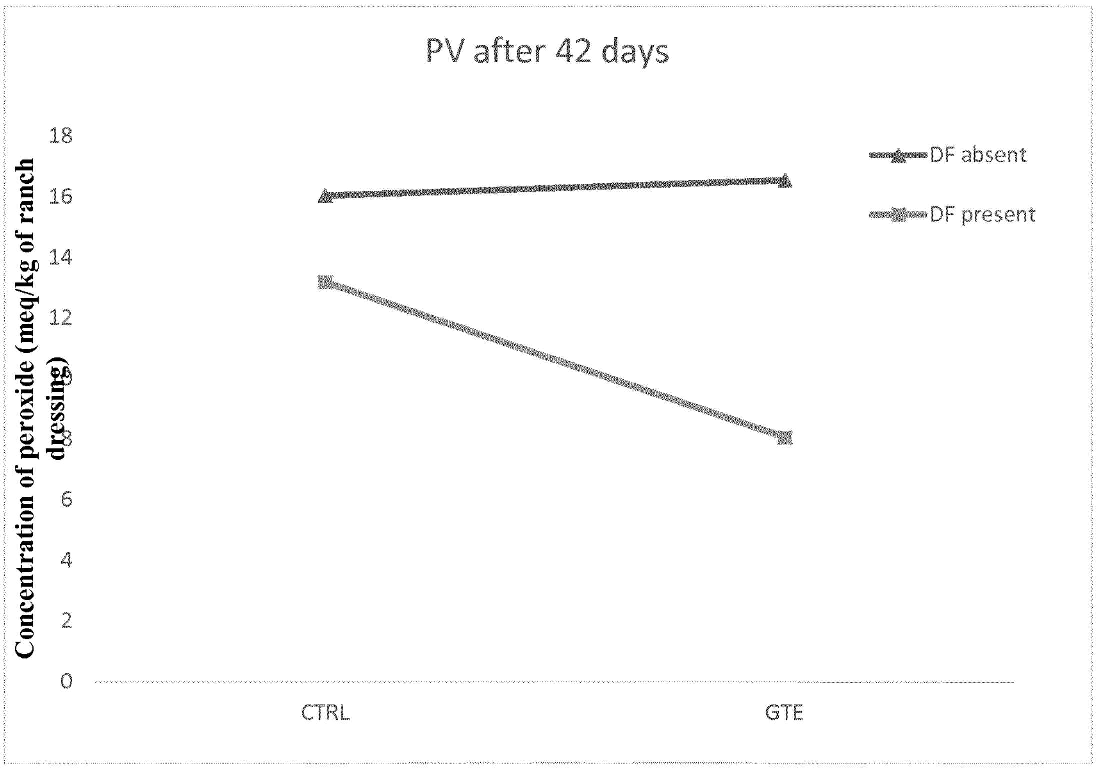
PV after 42 days
Concentration of peroxide (meq/kg of ranch dressing)
18
16
14
12
10
8
6
4
2
0
CTRL
GTE
DF absent
DF present

FIG. 3D

Europäisches Patentamt
European Patent Office
Office européen des brevets

# EUROPEAN SEARCH REPORT

Application Number
EP 19 16 0264

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/096050 A1 (INAOKA SETSUJIRO [JP] ET AL) 22 May 2003 (2003-05-22) * paragraph [0024] - paragraph [0026]; claims 1-16; tables 1,2 * | 1-3,5,6, 9-14 | INV. A23L27/00 A23L27/60 A23L33/105 |
| X | GB 2 478 967 A (INTERNAT HAIR COSMETICS [GB]) 28 September 2011 (2011-09-28) * claims 1-4,9-10,22 * | 1,2,4,6, 9 | |
| X | US 2006/003033 A1 (MCCLELLAN STEPHANIE N [US] ET AL) 5 January 2006 (2006-01-05) * paragraph [0051]; claims 1,2,9,11 * * paragraph [0035] * | 1,2,4-6, 9-11,15 | |
| X | PLATAT CARINE ET AL: "Production of functional pita bread using date seed powder", JOURNAL OF FOOD SCIENCE AND TECHNOLOGY, SPRINGER (INDIA) PRIVATE LTD, INDIA, vol. 52, no. 10, 28 January 2015 (2015-01-28), pages 6375-6384, XP035536139, ISSN: 0022-1155, DOI: 10.1007/S13197-015-1728-0 [retrieved on 2015-01-28] * abstract; tables 1,2 * * page 6383, left-hand column, paragraph 2 * | 1,2,7,8, 12,15 | TECHNICAL FIELDS SEARCHED (IPC): A23L |

-/--

The present search report has been drawn up for all claims

1

| Place of search | Date of completion of the search | Examiner |
|---|---|---|
| The Hague | 22 July 2019 | Kirchhoff, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 16 0264

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | MANZOOR AHMAD SHAH ET AL: "Plant extracts as natural antioxidants in meat and meat products", MEAT SCIENCE., vol. 98, no. 1, 24 April 2014 (2014-04-24), pages 21-33, XP055270591, GB ISSN: 0309-1740, DOI: 10.1016/j.meatsci.2014.03.020 * the whole document * * page 25, right-hand column, line 39 - line 47 * ----- | 1-15 | |
| A | SAJID MAQSOOD ET AL: "Retardation of quality changes in camel meat sausages by phenolic compounds and phenolic extracts : Phenolic Compounds as Natural Preservative in Camel Meat Sausages", ANIMAL SCIENCE JOURNAL - NIHON CHIKUSAN GAKKAIHO, vol. 87, no. 11, 10 March 2016 (2016-03-10), pages 1433-1442, XP055607078, JP ISSN: 1344-3941, DOI: 10.1111/asj.12607 * the whole document * ----- -/-- | 1-15 | TECHNICAL FIELDS SEARCHED (IPC) |

The present search report has been drawn up for all claims

1

| Place of search | Date of completion of the search | Examiner |
|---|---|---|
| The Hague | 22 July 2019 | Kirchhoff, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 16 0264

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SHAHDADI F ET AL: "Study of phenolic compound and antioxidant activity of date fruit as a function of ripening stages and drying process", JOURNAL OF FOOD SCIENCE AND TECHNOLOGY, SPRINGER (INDIA) PRIVATE LTD, INDIA, vol. 52, no. 3, 11 October 2013 (2013-10-11), pages 1814-1819, XP035461687, ISSN: 0022-1155, DOI: 10.1007/S13197-013-1177-6 [retrieved on 2013-10-11] * abstract * ----- | 1-15 | |
| A | SAMI GHNIMI ET AL: "Date fruit ( Phoenix dactylifera L.): An underutilized food seeking industrial valorization", NFS JOURNAL, vol. 6, 12 December 2016 (2016-12-12), pages 1-10, XP055606914, ISSN: 2352-3646, DOI: 10.1016/j.nfs.2016.12.001 * the whole document * ----- | 1-15 | |
| | | | TECHNICAL FIELDS SEARCHED (IPC) |

1

The present search report has been drawn up for all claims

| Place of search | Date of completion of the search | Examiner |
|---|---|---|
| The Hague | 22 July 2019 | Kirchhoff, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO.

EP 19 16 0264

This annex lists the patent family members relating to the patent documents cited in the above-mentioned European search report. The members are as contained in the European Patent Office EDP file on
The European Patent Office is in no way liable for these particulars which are merely given for the purpose of information.

22-07-2019

| Patent document cited in search report | Publication date | Patent family member(s) | Publication date |
|---|---|---|---|
| US 2003096050 A1 | 22-05-2003 | JP 3592681 B2 | 24-11-2004 |
| | | JP 2003033157 A | 04-02-2003 |
| | | US 2003096050 A1 | 22-05-2003 |
| GB 2478967 A | 28-09-2011 | GB 2478967 A | 28-09-2011 |
| | | WO 2011117642 A2 | 29-09-2011 |
| US 2006003033 A1 | 05-01-2006 | US 2006003033 A1 | 05-01-2006 |
| | | US 2010272832 A1 | 28-10-2010 |
| | | US 2010272833 A1 | 28-10-2010 |
| | | US 2011003020 A1 | 06-01-2011 |
| | | WO 2006004759 A2 | 12-01-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5096718 A


### Non-patent literature cited in the description


- a substance used to preserve food by retarding deterioration, rancidity, or discoloration due to oxidation. *Food and Drug Administration (21CFR 170.3)*
- *IDF (International Dairy Federation) Standards 74A,* 1991
- **KUBO I. ; MUROI H. ; HIMEJIMA M.** Antimicrobial activity of green tea flavour components and their combination effects. *Journal of Agricultural and Food Chemistry,* 1992, vol. 40, 245-248
- **YAM T. S. ; SHAH S. ; HAMILTON-MILLER J. M.** Microbiological activity of whole and fractionated crude extracts of tea (Camellia sinensis), and of tea components. *FEMS Microbiol. Lett.,* 1997, vol. 152, 169-174
- **KAJIYA K. ; KUMAZAWA S. ; NAKAYAMA T.** Steric effects on interaction of tea catechins with lipid bilayers. *Bioscience, Biotechnology and Biochemistry,* 2001, vol. 65, 2638-2643
- **GHNIMI S. ; UMER S. ; KARIM A. ; KAMAL-ELDIN A.** Date fruit (Phoenix dactylifera L.): An underutilized food seeking industrial valorization. *NFS Journal,* 2017, vol. 6, 1-10
- *IDF (International Dairy Federation) Standard Methods,* 1991